# EUROPEAN PATENT SPECIFICATION

(11) **EP 0 673 383 B1**
(45) Date of publication and mention of the grant of the patent: **31.05.2000**
(21) Application number: 92915188.4
(22) Date of filing: 18.06.1992
(51) Int. Cl.: C07K 14/59, A61K 38/24

(54) **ANALOGS OF GLYCOPROTEIN HORMONES HAVING ALTERED RECEPTOR BINDING SPECIFICITY AND ACTIVITY AND METHODS FOR PREPARING AND USING SAME**
ANALOGA VON GLYKOPROTEINHORMONEN MIT GEÄNDERTER REZEPTORBINDUNGSSPEZIFITÄT UND AKTIVITÄT UND VERFAHREN ZUR DARSTELLUNG UND VERWENDUNG DERSELBEN
ANALOGUES DES HORMONES GLYCOPROTEIQUES PRESENTANT UNE SPECIFICITE ET UNE ACTIVITE DE LIAISON AVEC LES RECEPTEURS MODIFIEES, ET PROCEDES DE PREPARATION ET D'UTILISATION DE CES ANALOGUES

(30) Priority: 18.06.1991 US 717151
(43) Date of publication of application: 27.09.1995
(73) Proprietor: UNIVERSITY OF MEDICINE & DENTISTRY OF NEW JERSEY, Newark, New Jersey 07107-3000 (US)
(72) Inventor: MOYLE, William, R., Piscataway, NJ 08854 (US); CAMPBELL, Robert, K., Wrentham, MA 02093 (US)
(74) Representative: VOSSIUS & PARTNER
(86) International application number: US9205207
(87) International publication number: WO9222568

(56) References cited:
- WO-A-90/09800
- WO-A-91/16922
- THE JOURNAL OF CELL BIOLOGY, vol. 109, 1989 pages 1429-1438, M.M. MATZUK ET AL. 'Mutagenesis and Chimeric Genes Define Determinants in the beta Subunits of Human Chorionic Gonadotropin and Lutropin for Secretion and Assembly'
- Endocrine Review, Volume 12, No. 1, issued 1991, B.D. MURPHY et al., "Equine Chorionic Gondotropin", pages 27-44.
- Proceedings of the National Academy of Sciences, Volume 88, issued February 1991, R.K. CAMPBELL et al., "Conversion of Human Chorioganadotropin into a Follitropin by Protein Engineering", pages 760-764.

## Description

### Field of the Invention

The present invention relates to glycoprotein hormone analogs having altered receptor binding specificity and activity. Specifically, the invention pertains to alpha, beta-heterodimeric polypeptides having binding affinity to follicle stimulating hormone (FSH) receptors and luteinizing hormone (LH) receptors. The residues important for receptor binding and specificity are located between Cys93 and Cys100 and Cys100 and Cys110 of hCG. These residues correspond to hFSH residues 87-94 and 94-104. A wide variety of alpha, beta-heterodimeric polypeptides can be made to alter the LH and FSH receptor binding activity and specificity of the polypeptides without disrupting their abilities to form alpha, beta-heterodimers or react with antibodies. This invention also pertains to methods for preparing these alpha, beta-heterodimeric polypeptides and the pharmaceutical compositions in which they may be employed.

### The Reproductive Glycoprotein Hormones and Their Biological Actions.

The glycoprotein hormone family consists of three alpha, beta heterodimeric glycoproteins found in the anterior pituitary gland where they are made and includes luteinizing hormone (LH), follicle stimulating hormone (FSH), and thyroid stimulating hormone (TSH). In some species, a glycoprotein hormone structurally similar to LH is found in the placenta wherein it is synthesized. In humans, this glycoprotein hormone is called human chorionic gonadotropin (hCG). In primates, significant quantities of all the hormones are also found as excretion products in urine. Urine from pregnant women serves as a convenient source of hCG. After menopause, when the secretion of LH and FSH from the anterior pituitary is greatly increased, significant quantities of LH and FSH are found in the urine and are termed human menopausal gonadotropins (hMG). Urine from menopausal women serves as an important source of LH and FSH activities. Both urinary hCG and hMG have important commercial uses. Unlike hCG, which interacts with LH receptors and only weakly with FSH receptors, hMG interacts with both LH and FSH receptors. The activity of hMG is due to the presence of multiple hormone species in the urinary extract.

Gonadotropins such as CG, LH, and FSH play a major role in the reproductive process while the structurally related hormone, TSH, is important for thyroid function. Both LH and FSH are essential for normal reproductive function and treatments to reduce LH activity result in infertility, termination of pregnancy, or both. FSH plays a crucial role in fertility. In the absence of sufficient FSH, women fail to have a normal menstrual cycle, i.e., a cycle in which a follicle develops to the point of ovulation. In polycystic ovarian disease, fertility can be restored by administration of FSH to women. The hormone hCG is important for maintenance of pregnancy. In males, the absence of LH and FSH is associated with infertility. The hormone LH is required for puberty and, in its absence, there is a failure to acquire the sexual attributes and fertility of an adult. The clinical activities of these hormones are reviewed extensively in several standard textbooks including that by Yen and Jaffe (1).

Both hCG and LH bind to luteinizing hormone receptors (LHR). In the testis, LHR are found primarily in the Leydig cells. In the ovary, LHR are found primarily in thecal, FSH-stimulated granulosa, and luteal cells. The major role of LH is to stimulate the formation of steroid hormones including the androgens testosterone and androstenedione (Leydig and thecal cells) and progesterone (FSH-stimulated granulosa, thecal, and luteal cells). LH also causes ovulation of mature follicles. While hCG is normally produced only by the placenta during pregnancy, due to its high affinity for LH receptors, the ease which it can be purified from urine, and its long biological half-life, hCG has been widely used as a substitute for LH. Important clinical uses for hCG include stimulation of fertility in males and induction of ovulation in females.

FSH binds to FSH receptors (FSHR) which are located primarily in the Sertoli cells of the testis and the granulosa cells of the ovaries. The primary roles of FSH are to stimulate the conversion of androgens (i.e., the steroids produced in response to LH stimulation of testicular Leydig cells and ovarian thecal cells) to estrogens, to promote the synthesis of inhibin and activin, to promote the development of Sertoli and granulosa cells, and to stimulate gamete maturation. The effect of FSH on granulosa cells leads to follicular maturation, a process during which the oocyte is prepared for ovulation and in which the granulosa cells acquire the ability to respond to LH. Follicle maturation is essential for the ability of LH to induce ovulation. Initiation of spermatogenesis requires FSH in addition to LH.

The differences in the effects of FSH and LH and the complex endocrine interactions between the two hormones cause them to have synergistic effects. For example, normal estrogen production is due to the effect of LH on androgen formation and the influence of FSH on the conversion of androgens to estradiol. This process is regulated by negative and positive feedback mechanisms wherein estradiol can inhibit FSH secretion and increase LH secretion from the pituitary gland. For this reason, the ratio of LH/FSH activity as well as the absolute hormone levels in blood are important for reproductive functions such as sperm production and ovulation of the proper number of oocytes during the menstrual and estrus cycles.

### Uses of Glycoprotein Hormones and Desirability of Novel Hormone Analogs.

Mixtures of FSH and LH activities (hMG) are routinely used in the treatment of human infertility, a condition affecting approximately 10% of all couples (2, 3). This particular combination therapy is necessary because gonadal support of gamete naturation is dependent upon the synergistic actions of both FSH and LH. Current treatment protocols requiring FSH and LH activity utilize urinary extracts from postmenopausal women. The use of these extracts is compromised by several factors including batch (4, 5) and supplier (6) variability, expense of treatment (7), and the risk of gonadal hyperstimulation, a potentially fatal side effect (8). These limitations potentially would be overcome through the use of hormone analogs which combine FSH and LH activity in the same molecule. Although there is a naturally occurring hormone, equine lutropin (eLH) which exhibits both FSH and LH activity in certain nonequine species (9), its practical application has been limited by side effects (8) and cross-species intolerance (3). Because the structural basis of the LH and FSH activity of eLH has not been understood, it has not been useful as a model for the engineering of mixed functions into hormones from other species. Further, it is not possible to reproducibly prepare eLH with different ratios of LH to FSH activity. In women with some forms of polycystic ovarian disease in which endogenous LH levels are elevated, it would be desirable to devise methods for producing glycoprotein hormone analogs which have a relatively higher ratio of FSH:LH activity. While protein engineering techniques have recently been reported to prepare analogs of hCG which bind with high affinity to FSH receptors (10), there has been no procedure for preparing glycoprotein hormones which bind well to LH and FSH receptors or which bind to LH and FSH receptors with a predictable ratio of FSH:LH activities.

Pathological changes in the ratios of FSH:LH are often associated with infertility (e.g., polycystic ovarian disease). Induction of ovulation can be influenced by the ratio of FSH:LH in serum (9) and it would be desirable to prepare analogs of glycoprotein hormones with any given ratio of LH/FSH activity. Presently, the only means of adjusting the ratios of LH to FSH in hormone preparations is to add FSH, LH, or hCG to them or to change the purification scheme. LH, FSH, and hCG have greatly differing half-lives and the ratio of hormone activity following in vivo administration of these preparations changes with time. Thus, mixtures of FSH and LH would gradually assume a higher ratio of FSH:LH activity following administration due to the longer half-life of FSH relative to LH. Since hCG has a much longer half-life than FSH, the ratio of FSH:LH activity of hFSH/hCG mixtures would gradually decrease after administration. It would be desirable to have analogs which contained a predefined ratio of LH to FSH activity in the same molecule. The ratio of FSH:LH activity in analogs with the capacity to bind to both LH and FSH receptors is expected to remain relatively constant after hormone administration. Analogs containing sequences derived from LH and FSH would have relatively short half-lives. In contrast, since the half-life of hCG is much longer than that of LH or FSH, if one were to use hCG as the primary structural component of these analogs, it should be possible to make analogs with a very long half-life. This would reduce the amounts of the analog needed for a biological effect. Small amounts of these analogs with approximately equal LH and FSH activity would be expected to be useful for inducing ovulation in women with hypothalamic amenorrhea and in males who fail to undergo puberty. Small amounts of analogs with lower ratios of LH/FSH activity would be expected to be useful clinically in cases where some endogenous LH is present such as inducing ovulation in women with polycystic ovarian disease or for increasing spermatogenesis in azospermic males who have some circulating LH. Previously, we have shown that it is possible to produce hCG analogs having very low LH activity and very high FSH activity (10). While these are primarily useful as FSH analogs, their LH activity could be increased only if they were mixed with hCG. Analogs of the human hormones with significant intrinsic LH and FSH activities have not yet been devised. In addition, based on the existing knowledge of the structure and functions of the glycoprotein hormones reviewed below, there is no obvious strategy which can enable these analogs to be devised.

### Structures of the Glycoprotein Hormones

The structures of the glycoprotein hormones have been studied for many years and considerable information exists as to the relative roles of the hormone subunits (11). These hormones share a common alpha-subunit but differ in their hormone-specific beta-subunits which determine the biological and immunological properties of each hormone. The sequences of the subunits were determined several years ago and were confirmed from the base sequences of the subunit cDNA which had been cloned from pituitary and placental libraries (12, 13). Substitution of the alpha-subunits of any one hormone for that of another does not change the receptor binding properties of the new hormone. Substitution of the beta-subunit is accompanied by a change in the receptor binding specificity of the resulting hormone. Thus, when FSH beta-subunit is substituted for the LH beta-subunit, the recombined hormone acquires the properties of FSH and loses those characteristics of LH.

Several attempts have been made to identify portions of the alpha- and beta-subunits of the hormones which are responsible for their unique biological properties. Earlier studies were based on chemical modifications of the hormones (11). Modifications were described which reduced the biological activities of the hormones but no analogs were prepared which had switched LH and FSH receptor binding specificities. Due to the complexity of the hormones, this approach was unable to identify amino acid residues which were involved in ligand binding specificity. In an attempt to simplify the problem of identifying residues which are involved in ligand binding specificity, some investigators prepared synthetic peptides corresponding to partial sequences of the alpha- or beta-subunits and monitored their abilities to inhibit binding of ¹²⁵I-hCG and ¹²⁵I-hFSH to LH and FSH receptors. Synthetic peptides corresponding to amino acid residues of hCG-beta 38-57 or hFSH-beta 31-52 appear to have higher abilities than most other peptides to bind to these receptors (14, 15, 16). However, they have extremely low affinities for the receptors, an observation which precludes their practical use.

A breakthrough in the ability to make and characterize glycoprotein hormone analogs came in 1985 when genetically engineered mammalian cells were first shown to express biologically active hCG heterodimers (17). Since that time several laboratories have used mammalian cells to express glycoprotein hormone analogs which are capable of binding to receptors and inducing a biological function (10, 18-21). These analogs appear to be glycosylated similar to the naturally occurring hormones. While not important for hormone-receptor interaction, glycosylation of the hormones has been shown to be important for signal transduction in many species (22). In these procedures one introduces a "gene" that encodes the desired amino acid sequences into mammalian cells downstream of a promoter. Construction of these genes is a standard recombinant DNA procedure wherein the codons of the genes encoding the alpha or beta-subunits of the hormones are changed to encode amino acid residues of the desired analogs using the well established genetic code (23, 24). When these gene constructs are transfected into mammalian cells using standard protocols (23, 24), they direct the secretion of glycosylated hormone analogs into the culture media. These media can be assayed for the presence of immunological or biological activity (10, 21).

Using mammalian cell expression systems to make hormone analogs, Campbell et al. (10) engineered an analog which converted hCG from a hormone which bound to LH receptors to an analog which bound to FSH receptors and had only slightly higher affinity for LH receptors than FSH. Campbell et al. (10) were unsuccessful in obtaining analogs which had a high affinity for both LH and FSH receptors. As noted earlier, some naturally occurring analogs (i.e., eLH) can bind to both LH and FSH receptors and this property suggested that it should be possible to engineer human glycoprotein hormone analogs with the abilities to bind to both LH and FSH receptors. None of these analogs have been reported as yet.

One of the most interesting findings made from the studies of Campbell et al. (10) was that the region of hCG and hFSH which appeared to be important for receptor interaction was different from that which had been presumed to interact with the LH and FSH receptors based on the results of studies using synthetic peptides. Indeed, Campbell et al. (10) found that the amino acid residues which had been thought to control receptor binding specificity on the basis of studies using synthetic peptides (i.e., amino acids 38-57 of hCG and 32-51 of hFSH) did not convey receptor binding activity in the intact hormone, the form which had highest affinity for the receptors.

### BRIEF DESCRIPTION OF THE FIGURES

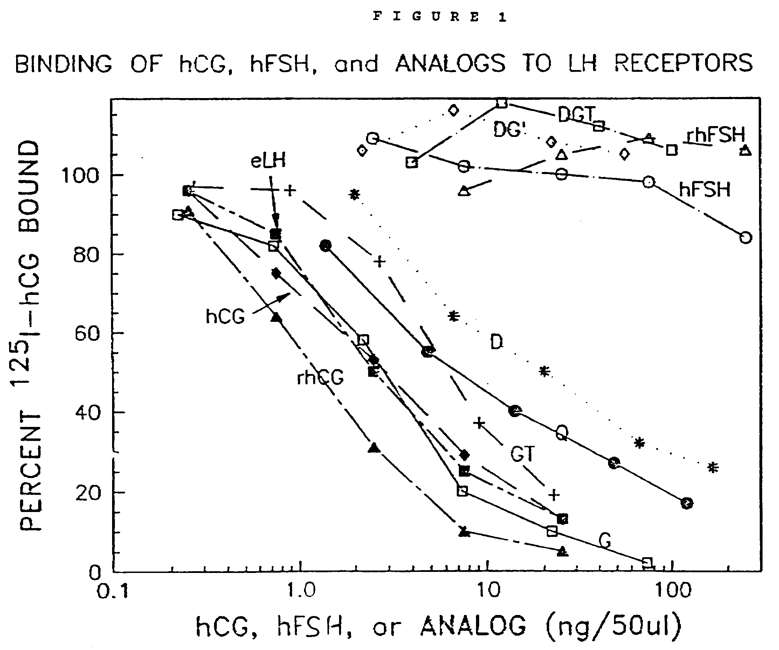

Figure 1 depicts in graphic format the ability of hCG, hFSH, and the alpha, beta-heterodimeric polypeptide analogs of the present invention to inhibit binding of ¹²⁵I-hCG to rat corpora luteal LH receptors.

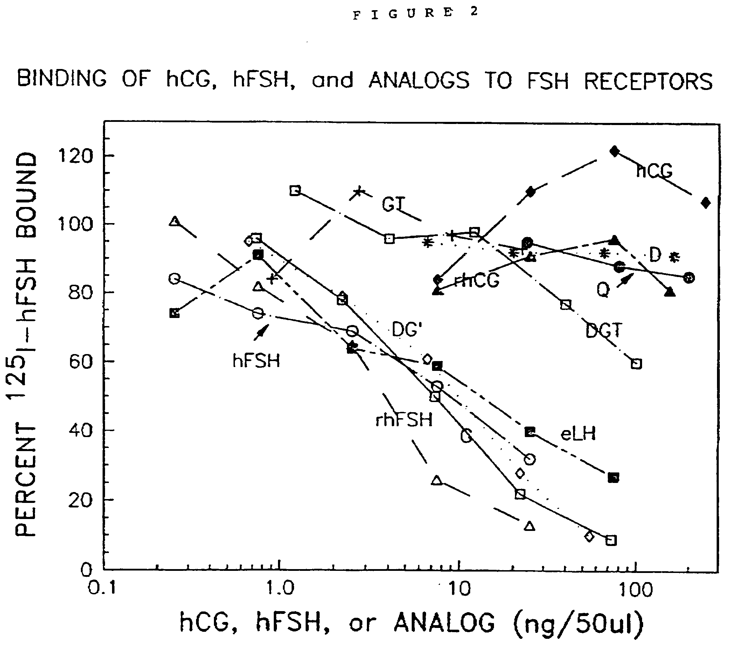

Figure 2 depicts in graphic format the ability of hCG, hFSH, and the alpha, beta-heterodimeric polypeptide analogs of the present invention to inhibit binding of ¹²⁵I-hFSH to bovine testes tissue homogenates.

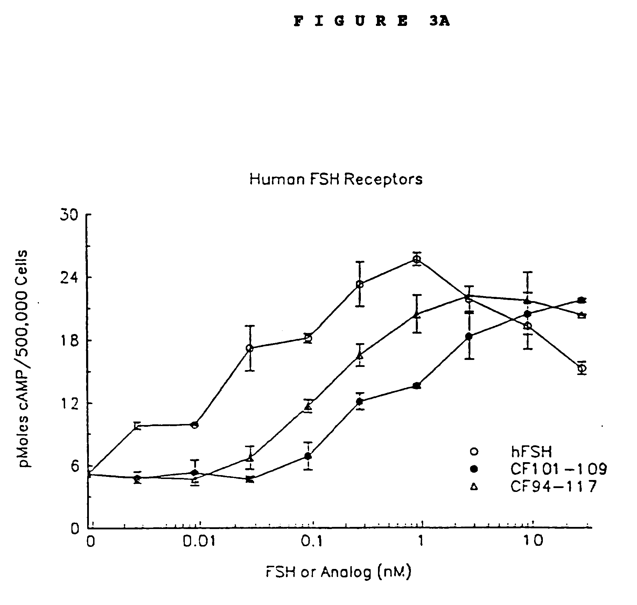

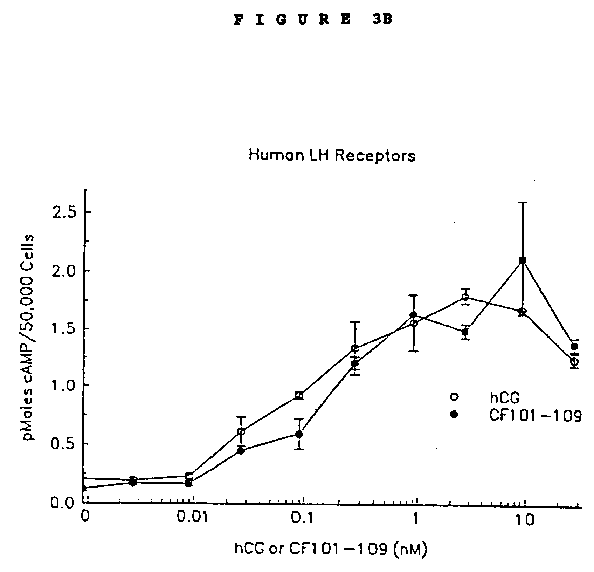

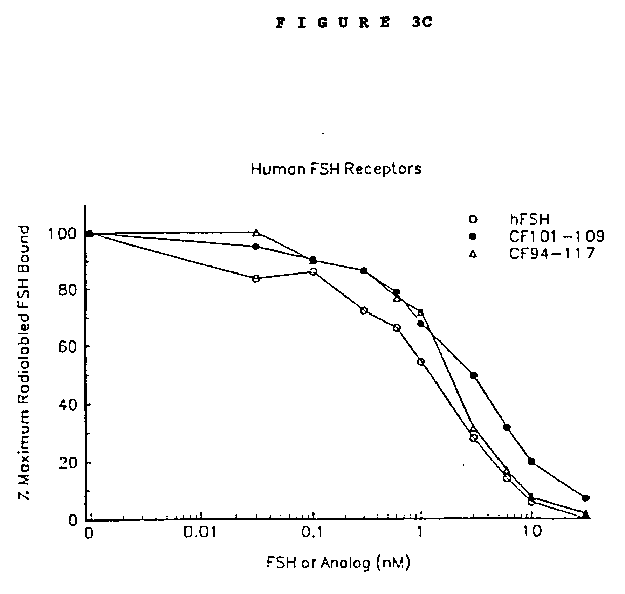

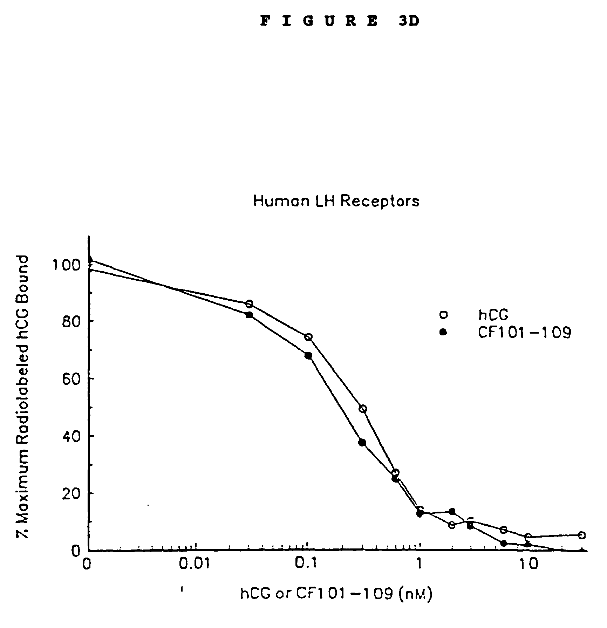

Figure 3 illustrates the effects of hFSH, CF101-109, and CF94-117 on hFSH receptor dependent responses (left panels) and hCG and CF101-109 on hLH receptor dependent responses (right panel). The hFSH, CF101-109, and CF94-117 used in these assays was produced by C127 cell lines that were stably expressing hFSH or the analogs.

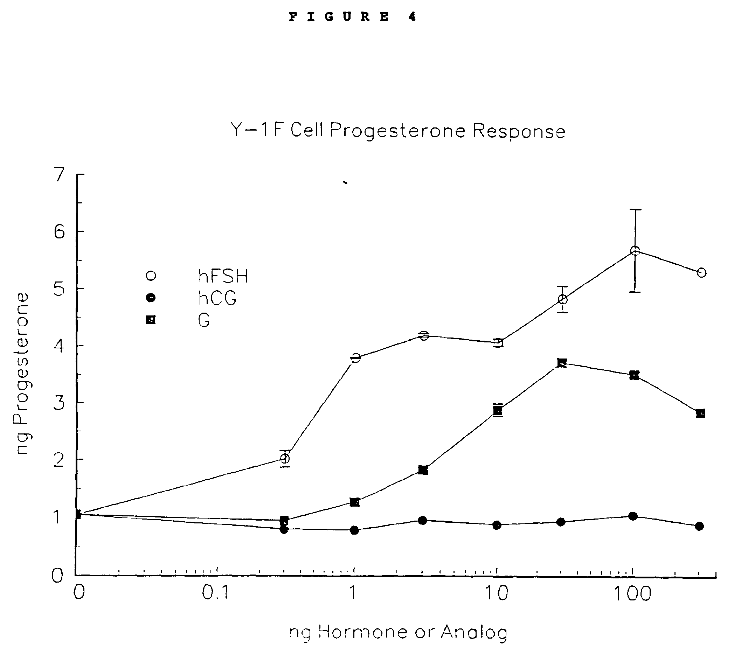

Figure 4 illustrates the effects of hFSH, hCG, and CF101-109 on a Y-1 adrenal cell line that was stably expressing the rat FSH receptor. Both hFSH and CF101-109 caused these cells to make progesterone (measured by radioimmunoassay) and round-up (detected by morphological observation). hCG was without effect on these assays. In other studies, hCG and CF101-109 caused rounding of Y-1 cells transfected with rat LH receptors. In the study shown here, Y-1F cells were added to a 24-well culture dish and grown until 60-70% confluent in F-12 medium containing 15% horse serum and 2.5% fetal bovine serum. Hormones were added at the amounts indicated in a total volume of 0.2 ml and incubation was continued for 4.5 hours at 37° C. The plates were examined for rounding at 30 minutes, one hour, and at the end of the study. Cells treated with hFSH and CF101-109 rounded. Cells treated with hCG did not round. At the end of the study, the cells and medium were frozen and saved for progesterone assay. The progesterone was determined by RIA using a commercial kit obtained from Leeco, Southfield Michigan.

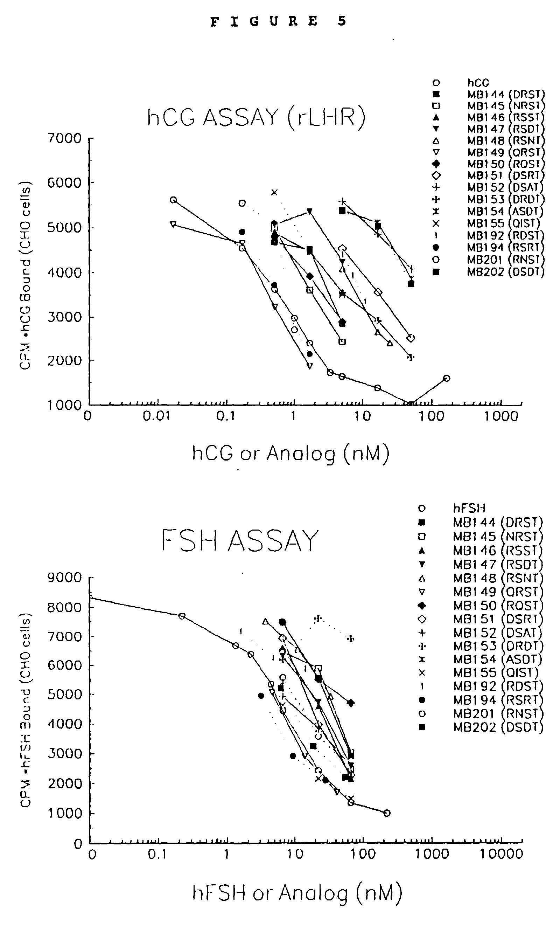

Figure 5 (TOP PANEL) illustrates the abilities of hCG and a series of CF101-109 analogs to bind to rat LH receptors expressed in CHO cells. Figure 5 (BOTTOM PANEL) illustrates the abilities of hFSH and the same series of CF101-109 analogs to bind to human FSH receptors expressed in CHO cells. The concentrations of the analogs were determined using A113/B105 sandwich immunoassays. In both panels the solid lines are associated with the top 12 symbols. The dotted lines are associated with the lowest 5 symbols.

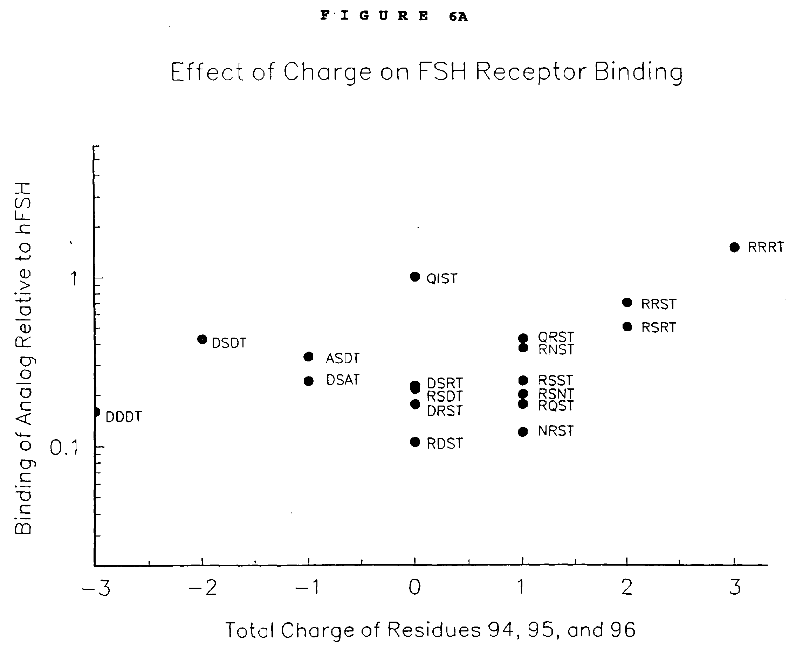

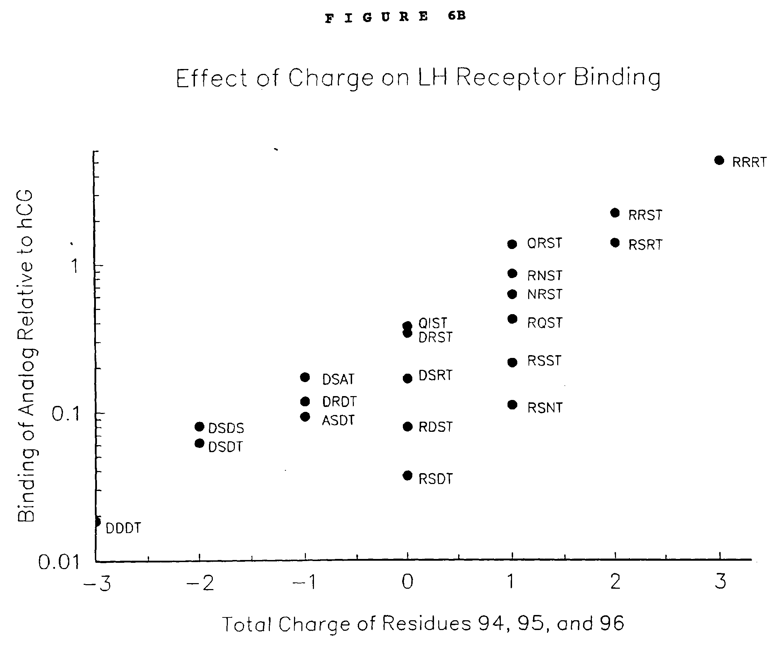

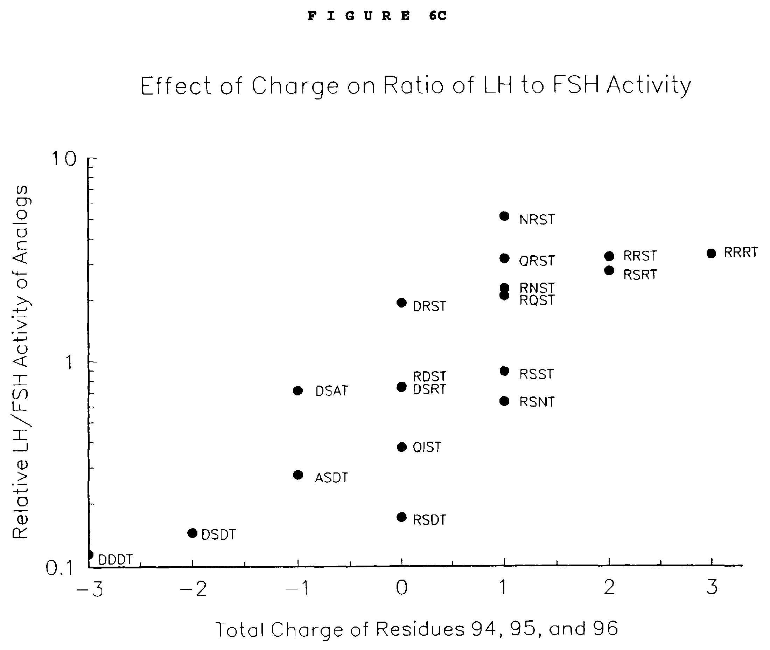

Figure 6A illustrates the abilities of CF101-109 analogs to bind to FSH receptors compared with that of hFSH. The values were obtained by comparing the abilities of the analogs and hFSH to inhibit binding of ¹²⁵I-hFSH to CHO cells expressing FSH receptors. Figure 6B illustrates the abilities of CF101-109 analogs to bind to LH receptors compared with that of hCG. The values were obtained by comparing the abilities of the analogs and hCG to inhibit binding of ¹²⁵I-hCG to CHO cells expressing LH receptors. Figure 6C illustrates the relative LH and FSH potencies of the CF101-109 analogs. The values were obtained by dividing the potencies of the analogs relative to hCG by the potencies of the analogs relative to FSH.

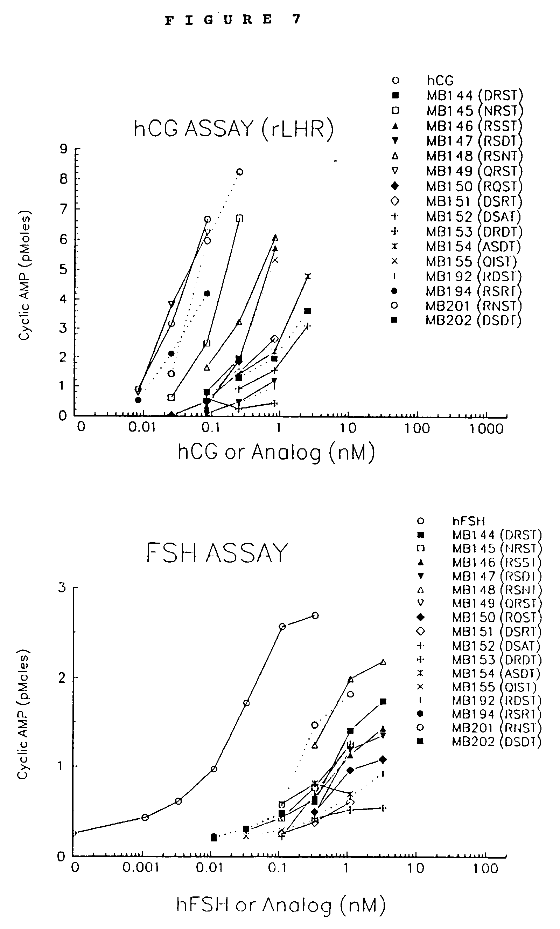

Figure 7 illustrates the abilities of hCG and a series of CF101-109 analogs to stimulate cyclic AMP formation from CHO cells stably expressing rat LH receptors (TOP PANEL) and the abilities of hFSH and the same series of CF101-109 analogs to stimulate cyclic AMP formation from CHO cells stably expressing human FSH (BOTTOM PANEL). The concentrations of the analogs were determined using A113/B105 sandwich immunoassays. In both panels the solid lines are associated with the top 12 symbols. The dotted lines are associated with the lowest 5 symbols.

### SUMMARY OF THE INVENTION

The present invention pertains to an alpha, beta-heterodimeric polypeptide having binding affinity to vertebrate luteinizing hormone (LH) receptors and vertebrate follicle stimulating hormone (FSH) receptors comprising a glycoprotein hormone alpha-subunit polypeptide and a non-naturally occurring beta-subunit polypeptide, wherein the beta-subunit polypeptide is a chain of amino acids comprising the following four joined subsequences:
(a) a first subsequence homologous to the amino acid sequence of residues 1-93 of the beta-subunit selected from the group consisting of human chorionic gonadotrophin (hCG), vertebrate luteinizing hormone (LH), vertebrate follicle stimulating hormone (FSH), and vertebrate thyroid stimulating hormone (TSH);
(b) a second subsequence homologous to the amino acid sequence of residues 94-97 of the beta-subunit selected from the group consisting of human chorionic gonadotrophin (hCG) and vertebrate luteinizing hormone (LH);
(c) a third subsequence homologous to the amino acid sequence of residues 98-100 of the beta-subunit selected from the group consisting of human chorionic gonadotrophin (hCG), vertebrate luteinizing hormone (LH), vertebrate follicle stimulating hormone (FSH), and vertebrate thyroid stimulating hormone (TSH); and
(d) a fourth subsequence homologous to the amino acid sequence of residues 101-110 of the beta-subunit of vertebrate follicle stimulating hormone.

### DETAILED DESCRIPTION OF THE INVENTION

The present invention relates to glycoprotein hormone analogs having altered receptor binding specificity and activity. Applicants have discovered that the residues important for receptor binding and specificity in glycoprotein hormones are located between Cys93 and Cys100 and Cys100 and Cys110 of hCG. These residues correspond to hFSH residues 87-94 and 94-104. A wide variety of alpha, beta-heterodimeric polypeptides can be made to alter the LH and FSH receptor binding activity and specificity of the polypeptides without disrupting their abilities to form alpha, beta-heterodimers or react with antibodies. The finding that interactions between regions of hCG beta-subunit amino acids 94-97 and 101-106 (corresponding to hFSH beta-subunit amino acids 88-91 and 95-100) play a dominant role in determining hormone-receptor binding specificity was unexpected because 1) Keutmann H.T. et al. (14), Santa Coloma and Reichert (15), and Schneyer, A.L., et al. (16) had shown that synthetic peptides containing beta-subunit amino acids from portions of the region 38-57 of hCG and 32-51 of hFSH were able to bind to LH and FSH receptors with low affinity and 2) Campbell et al. (10) had shown that residues 94-117 acted to cause an almost total shift in receptor binding specificity. The region containing amino acids 94-97 by itself did not influence receptor binding specificity and it was believed that modification of other residues in the 94-117 region of the molecule would also have an all-or-none effect on receptor binding specificity.

The alpha, beta-heterodimeric polypeptides of the present invention are "engineered" to alter the LH and FSH receptor binding activity and specificity of the polypeptides in vertebrates. Applicants have found that 1) the region of the hCG beta-subunit between 94-97 (i.e., "D" region) is most important for LH receptor binding activity and specificity, 2) the region of the hFSH beta-subunit between 100-106 (hCG numbering, i.e., "G" region) is most important for FSH binding activity and specificity and, 3) these regions ("D" and "G") of the beta-subunit are somewhat independent in activity. Substitution of a non-LH sequence in the "D" region will decrease binding of the polypeptide to the LH receptor. Substitution of a FSH sequence in the "G" region will increase binding of the polypeptide to the FSH receptor. Thus, it is possible to make modifications and substitutions in these regions to decrease the affinity of hCG for LH receptors and to dramatically increase the affinity of hCG for FSH receptors. The region of the beta-subunit between 1-93 and the choice of alpha-subunit does not appear to be important for LH and FSH binding activity and specificity. Hence analogs having a wide variety of activities can be made by altering the compositions of these two critical regions.

Generally, amino acid sequence variants will be substantially homologous with the relevant portion of the alpha, beta-heterodimeric polypeptides as set forth in Tables 1 and 2. Substantially homologous means that greater than about 70% of the primary amino acid sequence of the candidate polypeptide corresponds to the sequence of the relevant portion of the alpha, beta-heterodimeric polypeptide when aligned in order to maximize the number of amino acids residue matches between the two polypeptides. Alignment to maximize matches of residues includes shifting the amino and/or carboxyl terminus, introducing gaps as required or deleting residues present as inserts in the candidate polypeptide, or both. For example, see Tables 1 and 2 where the glycoprotein hormones and the alpha, beta-heterodimeric polypeptides are aligned for maximum homology. Typically, amino acid sequence variants will be greater than about 90% homologous with the corresponding sequences shown for the proteins in Tables 1 and 2.

Variants that are not hormonally-active fall within the scope of this invention, and include polypeptides that may or may not be substantially homologous with a sequence described herein but which are 1) immunologically cross-reactive with antibodies raised against the counterpart polypeptide or 2) capable of competing with such counterpart polypeptides for cell surface receptor binding. Hormonally active variants are produced by the recombinant or organic synthetic preparation of fragments or by introduction of amino acid sequence variations so that the molecule no longer demonstrates hormonal activity as defined herein.

Immunological or receptor cross-reactivity means that the candidate polypeptide is capable of competitively inhibiting the binding of the hormonally-active analogue to polyclonal antisera raised against the hormonally-active analogue, Such antisera are prepared in a conventional manner by injecting goats or rabbits S.C. with the hormonally active analogue or derivative in complete Freunds adjuvant, followed by booster intraperitoneal or S.C. injections in incomplete Freunds.

Variants that are not hormonally active but which are capable of cross-reacting with antisera to hormonally active polypeptides are useful a) as reagents in diagnostic assays for the analogues of their antibodies, b) when insolubilized in accord with known methods, as an agent for purifying anti-analogue anti-antibodies from anti-sera, and c) as an immunogen for raising antibodies to hormonally active analogues.

In accordance with the invention, the terminology "glycoprotein hormone" refers to human chorionic gonadotrophin (hCG), vertebrate luteinizing hormone (LH), vertebrate follicle stimulating hormone (FSH), and vertebrate thyroid stimulating hormone (TSH). The term "chimera" is used to designate a hormone analog which contains amino acid sequences derived from two or more different glycoprotein hormones.

In accord with the present invention, the alpha, beta-heterodimeric polypeptides comprise a glycoprotein hormone alpha-subunit polypeptide and a non-naturally occurring beta-subunit polypeptide. As set out above, the glycoprotein hormones share a common alpha-subunit. Substitution of the alpha-subunits of any one hormone for that of another does not significantly change the receptor binding properties of the new hormone. Accordingly, the alpha-subunit polypeptide in the present invention may be any vertebrate glycoprotein hormone alpha-subunit polypeptide. Nonlimiting examples of suitable alpha-subunit polypeptides include the alpha-subunits from human chorionic gonadotrophin (hCG), vertebrate luteinizing hormone (LH), vertebrate follicle stimulating hormone (FSH), and vertebrate thyroid stimulating hormone (TSH), and mixtures. In a preferred embodiment, the glycoprotein hormone alpha-subunit polypeptide may be selected from the group consisting of human chorionic gonadotrophin (hCG) and vertebrate luteinizing hormone (LH). In a more preferred embodiment, the glycoprotein hormone alpha-subunit polypeptide is human chorionic gonadotrophin (hCG). Preferably the glycoprotein hormone alpha-subunit polypeptide is a human polypeptide.

The beta-subunit of the alpha, beta-heterodimeric polypeptide is a non-naturally occurring beta-subunit polypeptide which is a chain of amino acids comprising four joined subsequences. The four joined subsequences are as follows:
(a) a first subsequence homologous to the amino acid sequence of residues 1-93 of the beta-subunit selected from the group consisting of human chorionic gonadotrophin (hCG), vertebrate luteinizing hormone (LH), vertebrate follicle stimulating hormone (FSH), and vertebrate thyroid stimulating hormone (TSH);
(b) a second subsequence homologous to the amino acid sequence of residues 94-97 of the beta-subunit selected from the group consisting of human chorionic gonadotrophin (hCG) and vertebrate luteinizing hormone (LH);
(c) a third subsequence homologous to the amino acid sequence of residues 98-100 of the beta-subunit selected from the group consisting of human chorionic gonadotrophin (hCG), vertebrate luteinizing hormone (LH), vertebrate follicle stimulating hormone (FSH), and vertebrate thyroid stimulating hormone (TSH);
(d) a fourth subsequence homologous to the amino acid sequence of residues 101-110 of the beta-subunit of vertebrate follicle stimulating hormone.

In one embodiment, the invention is directed to an alpha, beta-heterodimeric polypeptide having greater binding affinity for vertebrate follicle stimulating hormone (FSH) receptors than for vertebrate luteinizing hormone (LH) receptors comprising a glycoprotein hormone alpha-subunit polypeptide and a non-naturally occurring beta-subunit polypeptide, wherein the beta-subunit polypeptide is a chain of amino acids comprising the following four joined subsequences:
(a) a first subsequence homologous to the amino acid sequence of residues 1-93 of the beta-subunit selected from the group consisting of human chorionic gonadotrophin (hCG), vertebrate luteinizing hormone (LH), vertebrate follicle stimulating hormone (FSH), and vertebrate thyroid stimulating hormone (TSH);
(b) a second subsequence comprising 4 amino acids for residues 94-97;
(c) a third subsequence comprising 3 amino acids for residues 98-100; and
(d) a fourth subsequence homologous to the amino acid sequence of residues 101-110 of the beta-subunit of vertebrate follicle stimulating hormone.

In another embodiment, the invention is directed to an alpha, beta-heterodimeric polypeptide having binding affinity to follicle stimulating hormone (FSH) receptors and luteinizing hormone (LH) receptors comprising a glycoprotein hormone alpha-subunit polypeptide and a non-naturally occurring beta-subunit polypeptide, wherein the beta-subunit polypeptide is a chimera comprised of amino acids 1-100 of any vertebrate glycoprotein hormone homologous to amino acids found in residues 1-100 of human chorionic gonadotropin and any 1-20 amino acids which binds LH receptors better than FSH receptors and has biological activity.

In a preferred embodiment, the alpha, beta-heterodimeric polypeptide is selected from the group consisting of "G", "DG'", "Q", "D", "GT", and "DGT" as set out in Tables 1 and 2. In a more preferred embodiment, the alpha, beta-heterodimeric polypeptide is

Table 1 is a representation of the alpha, beta-heterodimeric polypeptides of the present invention using the one letter code and aligned in order to maximize the number of amino acids residue matches between the two polypeptides. Table 2 is a representation of the alpha, beta-heterodimeric polypeptides of the present invention using the three letter code. Abbreviations: A, Ala; C, Cys; D, Asp; E, Glu; F, Phe, G, Gly; H, His; I, Ile; K, Lys; L, Leu; M, Met; N, Asn; P, Pro; Q, Gln; R, Arg; S, Ser; T, Thr; V, Val; W, Trp; Y, Tyr.

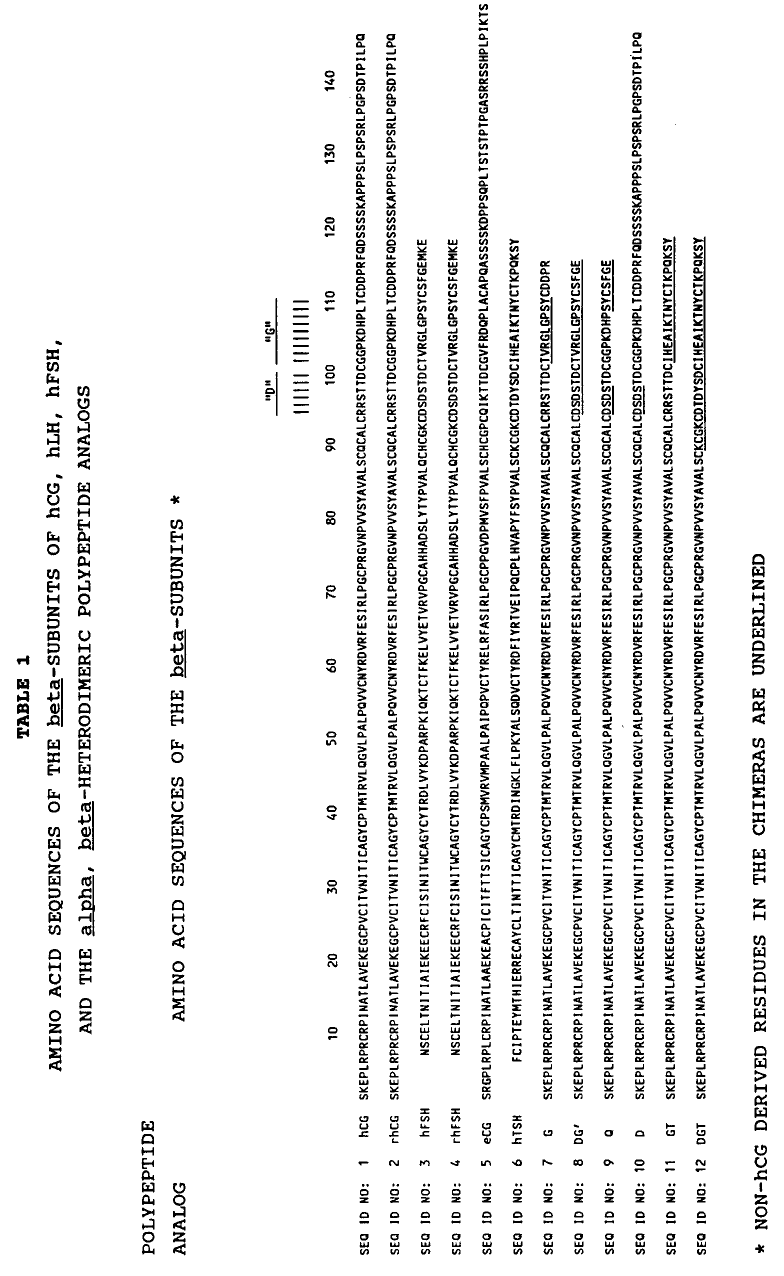

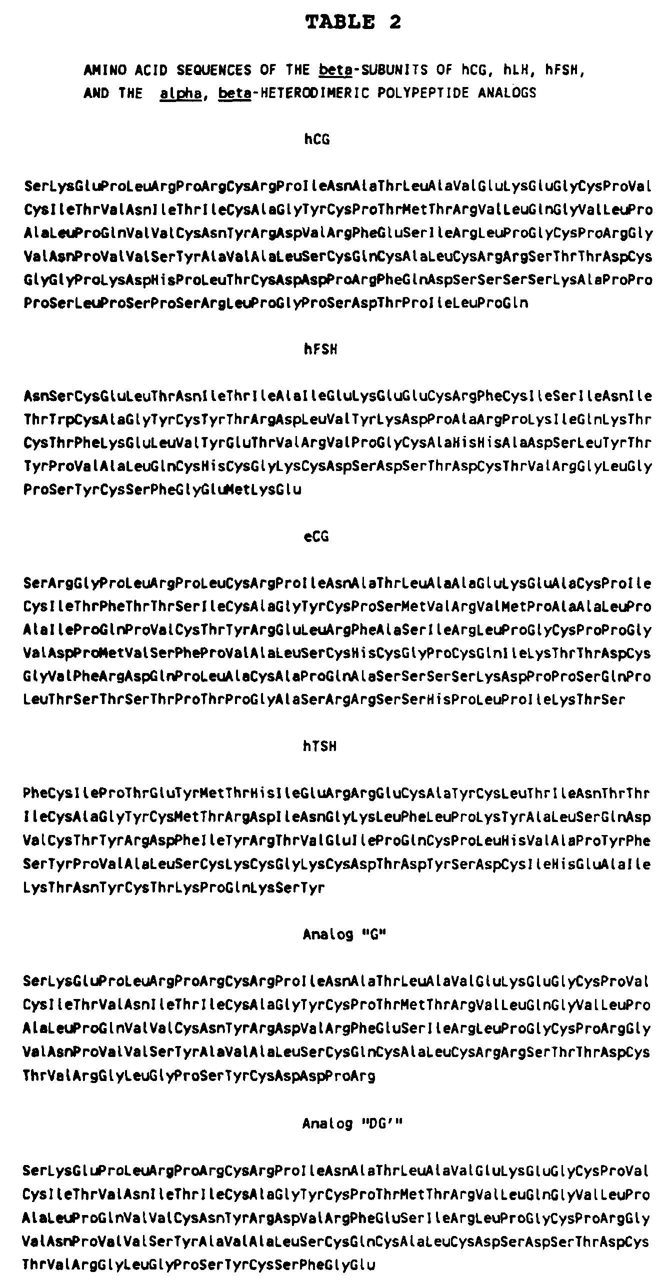

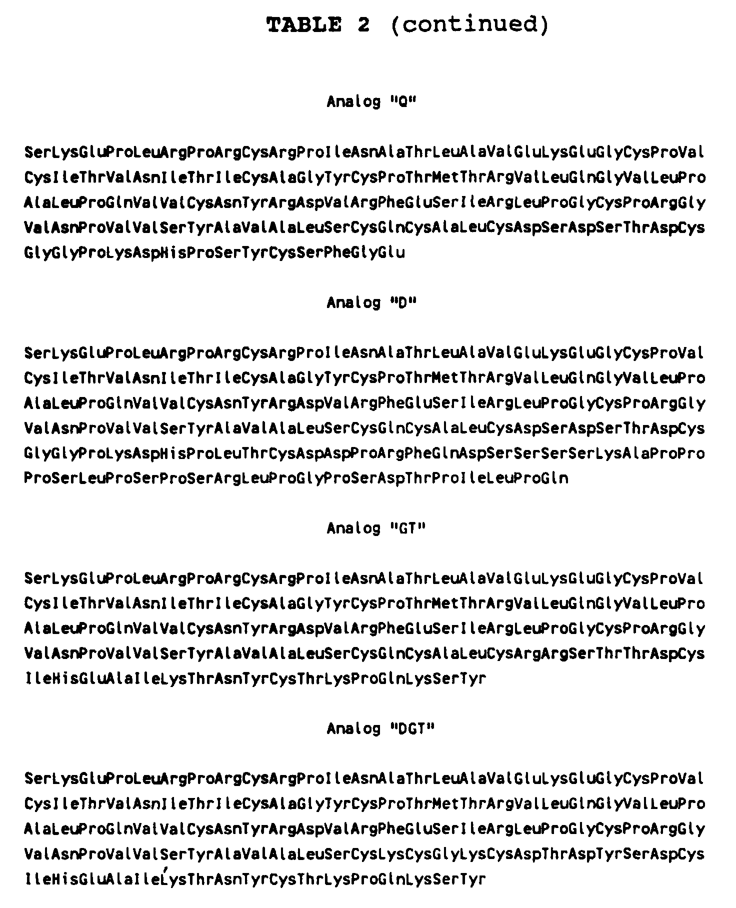

The activities of alpha, beta-heterodimeric polypeptide analogs "DG'," "Q," "D," "GT," and "DGT" are illustrated in Table 3, Figure 1, and Figure 2 along with hCG, hFSH, and eLH controls. The properties of some of these analogs have been described previously (10).

**Table 3**

| ACTIVITIES OF THE alpha, beta-HETERODIMERIC POLYPEPTIDE ANALOGS RELATIVE TO THAT OF hCG AND hFSH. | | |
|---|---|---|
| ANALOG | LH RECEPTORS | FSH RECEPTORS |
| rhCG | 1.0 | N.D. |
| rhFSH | N.D. | 1.0 |
| eCG | 0.53 | 0.18 |
| G | 0.62 | 0.29 |
| DG' | N.D. | 0.32 |
| Q | 0.15 | N.D. |
| D | 0.065 | N.D. |
| GT | 0.33 | N.D. |
| DGT | N.D. | N.D. |

These values were obtained by dividing the concentration of analog required to inhibit ¹²⁵I-hCG binding to rat luteal ovarian LH receptors or ¹²⁵I-hFSH binding to bovine testes FSH receptors by 50% into that required for recombinant hCG and recombinant hFSH, respectively. The concentrations of the analogs were determined by sandwich immunoassay using antibodies B105 and A113. The term "N.D." means not determined because concentrations of analog were not employed high enough to detect 50% inhibition. This was because the amounts of hFSH needed to bind to LH Receptors are several orders of magnitude greater than that of hCG and vice versa the amounts of hCG needed to bind to FSH receptors are several orders of magnitude greater than that of hFSH.

These results show that it is possible to control the ratio of FSH:LH activity by modulating residues corresponding to hCG amino acids 94-99 and 101-109, hFSH amino acids 88-93 and 95-103, or hTSH amino acids 89-94 and 96-104. These regions are termed "D" and "G", respectively (c.f., Table 1). In general, substitution of a non-LH sequence in the "D" region decreases binding of the polypeptide to the LH receptor while substitution of a FSH sequence in the "G" region increases binding of the polypeptide to the FSH receptor. Analogs containing both "D" and "G" from hCG bind to LH receptors like hCG (10). Analogs containing both "D" and "G" from hFSH bind to FSH receptors like FSH (10). Analogs containing "D" from hCG and "G" from hFSH bind well to both LH and FSH receptors (Table 3, Figure 1, and Figure 2). Analogs containing "D" from hFSH and "G" from hCG bind to LH receptors with considerably lower potency than hCG (10, Table 3, Figure 1, and Figure 2) and do not bind to FSH receptors. Analogs with "D" from hCG and "G" from TSH (i.e., "GT") bind to LH receptors but not to FSH receptors (Table 3, Figure 1, and Figure 2). Analogs with "D" and "G" from hTSH do not bind very well to either LH or FSH receptors (Table 3, Figure 1, and Figure 2).

These results show that it is possible to convert a human hormone into a molecule which binds to both LH and FSH receptors by using the "D" region from hCG and the "G" region from hFSH. Since replacement of the "D" region of hCG by residues from the "D" region of hFSH or hTSH dramatically lowers the ability of the analog to bind to LH receptors (i.e., compare hCG with analog "D," analog "G" with analog "DG'," and analog "GT" with analog "DGT"), it is clear that hFSH or hTSH substitutions in the "D" region which will make the analog less like hCG and will decrease its ability to bind to LH receptors. If the "G" region in these analogs is derived from hFSH, they will bind well to FSH receptors (i.e., compare analog "G" with analog "DG'"). Thus, as the residues in the "D" region are changed from those of hCG or any other hormone which binds to LH receptors to those of hFSH or hTSH, the ratios of LH:FSH activities will decrease and the analog will appear to behave more like FSH.

While the "D" region appears to have the greatest influence on binding of the analogs to LH receptors, the "G" region appears to have the greatest influence on binding of the analogs to FSH receptors. Thus, analog "G" binds FSH receptors better than analog "GT." Similarly, analog "DG'" binds FSH receptors better than analog "D" (Table 3, Figure 1, and Figure 2). Thus, as the residues in the "G" region are changed from hFSH or any other hormone which binds to FSH receptors to those of hCG or hTSH, the molecule will lose its ability to bind to FSH receptors, the ratios of LH:FSH activities will increase, and the analog will appear to behave more like hCG or LH.

By systematically varying the composition of residues in the "D" and "G" region between those of hCG, hFSH, and hTSH, it is possible to create a hormone analog which has any desired ratio of LH:FSH activity. Other regions of the hormone do not appear to have significant influence on receptor binding specificity (10).

Certain data suggest that the minimum sizes of regions "D" and "G" may be smaller than the sizes indicated. Thus in region "D" there are only 4 amino acids which differ between hCG and hFSH. Of these amino acids, only three are highly non-conserved (i.e., RRS/DSD). In region "G" there are 8 amino acids different between hCG and hFSH, however, it is clear that only 6 of these amino acid residues play an important role in receptor binding specificity. Thus, analog "Q" retains its ability to bind to LH receptors (Table 3, Figure 1, and Figure 2) even though it has two amino acid residues (i.e., 108 and 109) which are specifically found in the "G" region of hFSH, not hCG. On this basis, only 6 residues are believed to be critical in the "G" region.

The compounds of the present invention while effective in the form of the free form may be formulated and administered in the form of the therapeutically or pharmaceutically acceptable acid addition salts for purposes of stability, convenience of crystallization, increased solubility and the like. These acid addition salts include inorganic acid salts such as hydrochloric, hydrobromic, sulfuric, nitric, phosphoric, perchloric acid salts and the like; and organic acid salts such as acetic, trifluoroacetic, propionic, oxalic, hydroxyacetic, methoxyacetic, 2-hydroxypropanoic, 2-oxopropanoic, propanedioic, 2-hydroxy-butanedioic, benzoic, 2-hydroxybenzoic, 4-amino-2-hydroxybenzoic, 3-phenyl-2-propenoic, alpha-hydroxybenzeneacetic, methanesulfonic, ethanesulfonic, benzenesulfonic, toluenesulfonic, cyclohexanesulfamic, succinic, tartaric, citric, maleic, fumaric acid salts and the like. The preferred acid addition salts are chloride, oxalate and citrate. These acid addition salts can be prepared by conventional methods, such as by treatment of the free base of the inventive compound with the appropriate acid.

The compounds of the present invention, prepared in the free form, can be combined with a pharmaceutically acceptable carrier to provide a pharmaceutical composition. Suitable carriers for the free bases include propylene glycol-alcohol-water, isotonic water, sterile water for injection (USP), emulphor™-alcohol-water, cremophor-EL™ or other suitable carriers known to those skilled in the art.

The compounds of the present invention, prepared in the pharmaceutically acceptable acid addition salt form, can also be combined with a pharmaceutically acceptable carrier to provide a pharmaceutical composition. Suitable carriers for the acid addition salts include isotonic water, sterile water for injection (USP), alone or in combination with other solubilizing agents such as ethanol, propylene glycol, or other conventional solubilizing agents known to those skilled in the art.

Of course, the type of carrier will vary depending upon the mode of administration desired for the pharmaceutical composition as is conventional in the art. A preferred carrier is an isotonic aqueous solution of the inventive compound.

The compounds of the present invention can be administered to mammals, e.g., animals or humans, in amounts effective to provide the desired therapeutic effect. Since the activity of the compounds and the degree of the desired therapeutic effect vary, the dosage level of the compound employed will also vary. The actual dosage administered will also be determined by such generally recognized factors as the body weight of the patient and the individual hypersensitiveness of the particular patient. Thus, the unit dosage for a particular patient (man) can be as low as about 0.00005 mg/kg, which the practitioner may titrate to the desired effect.

The compounds of the present invention can be administered parenterally, in the form of sterile solutions or suspensions, such as intravenously, intramuscularly or subcutaneously in the carriers previously described. The compounds may also be administered orally, in the form of pills, tablets, capsules, troches, and the like, as well as sublingually, rectally, or transcutaneously with a suitable pharmaceutically acceptable carrier for that particular mode of administration as is conventional in the art.

For parental therapeutic administration, the compounds of the present invention may be incorporated into a sterile solution or suspension. These preparations should contain at least about 0.1% of the inventive compound, by weight, but this amount may be varied to between about 0.1% and about 50% of the inventive compound, by weight of the parental composition. The exact amount of the inventive compound present in such compositions is such that a suitable dosage level will be obtained. Preferred compositions and preparations according to the present invention are prepared so that a paranteral dosage unit contains from between about 0.5 milligrams to about 100 milligrams of the inventive compound.

The sterile solutions or suspensions may also include the following adjuvants: a sterile diluent, such as water for injection, saline solution, fixed oils, polyethylene glycol, glycerine, propylene glycol, or other synthetic solvent; antibacterial agents, such as benzyl alcohol or methyl paraben; antioxidants, such as ascorbic acid or sodium metabisulfite; chelating agents, such as ethylenediaminetetraacetic acid (EDTA); butters, such as acetates, citrates or phosphates; and agents for the adjustment of tonicity, such as sodium chloride or dextrose. The parental preparations may be enclosed in ampules, disposable syringes, or multiple dose vials made of glass or plastic.

The compounds of the present invention can also be administered orally. For oral therapeutic administration, the compounds may be incorporated with excipients and used in the form of tablets, capsules, elixirs, suspensions, syrups, wafers, chewing gums and the like. These preparations should contain at least about 4% of the inventive compound, by weight, but this amount may be varied depending upon the particular dosage form from between about 4% to about 70% of the inventive compound, by weight of the oral composition. The exact amount of the compound present in the composition is such that a suitable dosage will be obtained. Preferred compositions and preparations according to the present invention are prepared so that an oral dosage unit form contains from between about 5 to about 300 milligrams of the inventive compound.

The tablets, pills, capsules, troches and the like may also contain the following adjuvants: a binder, such as microcrystalline cellulose, gum tragacanth or gelatine; an excipient, such as starch or lactose; a disintegrating agent, such as alginic acid, Primogel, corn starch and the like; a lubricating agent, such as magnesium stearate or Sterotex; a gliding agent, such as colloidal silicon dioxide; a sweetening agent, such as sucrose or saccharin; and a flavoring agent, such as peppermint, methyl salicylate or orange flavoring. When the dosage form is a capsule, it may additionally contain a liquid carrier such as a fatty oil. Other dosage unit forms may contain other materials which modify the physical form of the dosage unit, such as enteric coatings. Thus tablets or pills may be coated with sugar, shellac, or other enteric coating agents. A syrup may contain, in addition to the above adjuvants, sucrose as a sweetening agent, preservatives, dyes, coloring agents and flavoring agents.

It is especially advantageous to formulate the pharmaceutical compositions in dosage unit forms for ease of administration and uniformity of dosage. The term dosage unit forms as used herein refers to physically discrete units suitable for use as a unitary dosage, each unit containing a predetermined quantity of active ingredient calculated to produce the desired therapeutic effect in association with the pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

Throughout this application, various publications have been referenced. The disclosures in these publications are incorporated herein by reference in order to more fully describe the state of the art.

The present invention is further illustrated by the following examples which are not intended to limit the effective scope of the claims. All parts and percentages in the examples and throughout the specification and claims are by weight of the final composition unless otherwise specified.

### EXAMPLES

### Preparation of "GT"

pSVLβ' was prepared by subcloning of the XhoI-BamHI fragment of pKBM-hCGβ' vector (10) into the XhoI-BamHI sites of pSVL (obtained from Pharmacia Co., Piscataway, NJ). The approximately 3.6-3.7Kbp PvuII-SstI fragment from pSVL-hCGβ' was ligated with the synthetic oligonucleotide pair:

C Q C A L C R R S T T D C I H E A I K T N Y C T K P Q K S Y *

5'-

CTGTCAATGTGCACTCTGCCGCAGATCTACCACTGACTGCATACATGAAGCCATCAAGACAAACTACTGCACC[?]   [?]CTCAGAAGTCCTACTGAA

GGCAGGTGAGCT-3'

and

3'-

GACAGTTACACGTGAGACGGCGTCTAGATGGTGACTGACGTATGTACTTCGGTAGTTCTGTTTGATGACGTGGTTTGGAGTCTTCAGGATGACTT

CCGTCCAC-5'.

An aliquot of the ligation mixture was taken and used to transform DH5-alpha strain E. coli. (obtained from Bethesda Research Laboratories, Gaithersburg, MD). Plasmid DNAs from ampicillin-resistant DH5-alpha clones were screened by digestion with BglII (which is unique to vectors containing the cassette) and EcoRI (which cuts in the vector). Positive clones were identified by the presence of two fragments (approximately 0.8Kbp and 2.9Kbp). The sequence in the coding region of one of these plasmids, which lacked most of the beta-subunit cDNA due to excision of the PvuII fragment, was confirmed by dideoxysequencing as described (10). The remainder of the beta-subunit cDNA (encoding hCGβ amino acids 1-87) was restored by ligation of the 2.3Kbp PvuI-PvuII fragment of this vector and the 2.9Kbp PvuI-PvuII fragment from pSVL-hCG-beta'. The ligation mixture was used to transform DH5-alpha strain E. coli. and ampicillin resistant clones were obtained. Miniprep plasmid DNA from these clones were digested with EcoRI and BglII, and DNA from positive clones exhibited fragments of approximately 2.5Kbp and 2.9Kbp. After the DNA was subjected to a dideoxy sequencing procedure to confirm that it encoded "GT" (Table 1), the plasmid DNA was then cotransfected into COS-7 cells (obtained from the American Type Culture Collection) along with pSVL-hCG-alpha, a pSVL-based plasmid encoding the human glycoprotein hormone alpha-subunit (10, 21), using a DEAE-dextran procedure (10). Beginning in 1-2 days and for a few days thereafter, the COS-7 cells produced significant amounts of the free subunits and the heterodimer. These were present in the culture media and heterodimer was detected using sandwich immunoassays employing monoclonal antibodies A113 and B105 (10). The protein was concentrated by ultrafiltration and monitored for its abilities to bind to LH and FSH receptors by radioligand receptor assays using ¹²⁵I-hCG and ¹²⁵I-hFSH as tracers and rat ovarian corpora lutea and bovine testes as sources of LH and FSH receptors as described (10).

### Preparation of "G"

We have found that an alpha, beta-heterodimer composed of the alpha-subunit of hCG and an hCG/hFSH beta-subunit chimera termed "G" having the amino acid sequence illustrated in Table 1 has high affinity for LH and FSH receptors as shown by its ability to compete with radiolabeled hCG and/or hFSH for binding to these receptors (Table 3, Figures 1 and 2). This analog can be prepared in a variety of methods well-known to one versed in the art of molecular biology, one of which is described here. The cDNA for analog "GT' was digested with BglII and SstI and the 5.2-5.3Kbp fragment was ligated with the oligonucleotides: 
R S T T D C T V R G L G P S Y C D D P R *

5'-GATCTACCACTGACTGCACCGTGAGAGGCCTCGGGCCCTCTTACTGCGATGACCCGCGGTAGAGCT-3'

and

3'-ATGGTGACTGACGTGGCACTCTCCGGAGCCCGGGAGAATGACGCTACTGGGCGCCATC-5'
 using standard methods (23, 24). The ligation mixture was used to transform competent DH5-alpha strain E. coli. (23, 24). Transformed cells were selected by their abilities to grow on agar plates containing amphicillin. Ampicillin resistant colonies were chosen and plasmid minipreparations were made by the boiling lysis method (23, 24). The plasmid DNA was then tested for the presence of HindIII-ApaI endonuclease restriction sites. Plasmid DNA having the desired sequences was cleaved into three fragments (approximately 0.8Kbp, 1.1Kbp, and 3.4Kbp). After the DNA was subjected to a dideoxy sequencing procedure to confirm that it encoded "G" (Table 1), the plasmid DNA was then cotransfected into COS-7 cells (obtained from the American Type Culture Collection) along with pSVL-hCG-alpha, a pSVL-based plasmid encoding the alpha-subunit (10, 21), using a DEAE-dextran procedure (10, 21, 23, 24). Beginning in 1-2 days and for a few days thereafter, the COS-7 cells produced significant amounts of the free subunits and the heterodimer. These were present in the culture media and heterodimer was detected using sandwich immunoassays employing monoclonal antibodies A113 and B105 (10). The protein was concentrated by ultrafiltration and monitored for its abilities to bind to LH and FSH receptors by radioligand receptor assays using ¹²⁵I-hCG and ¹²⁵I-hFSH as tracers and rat ovarian corpora lutea and bovine testes as sources of LH and FSH receptors as described (10).

### Preparation of "D" and "DG'" and "Q"

Preparations of analogs "D" and "DG'" have been described previously (10) and are the same as those of analogs CF94-97 and CF94-114 in that report, respectively. Analog "Q" was prepared from the expression vector encoding analogs "D" and CF108-114 (10) by digesting them with PpuMI, separating the fragments on agarose gels, and ligating the large fragment obtained from CF108-114 with the small fragment obtained from "D." The resulting plasmid was then cotransfected into COS-7 cells along with pSVL-hCG-alpha and the media assayed for the presence of the analogs using an A113-B105 sandwich immunoassay as described (10).

### Preparation of "DGT"

Plasmid pSVL-hCGβ' was sequentially digested with SstI and PvuII and the 3.6Kbp fragment was ligated with the synthetic DNA cassette formed by annealing the following oligonucleotides:

C K C G K C N T D Y S D C I H E A I K T N T C T K P Q K S Y *
5'-
CTGTAAGTGTGGCAAGTGCAATACTGACTACAGTGACTGTATACATGAAGCCATCAAGACAAACTACTGCACCAAACCTCAGAAGTCCTACTGAA
GGCAGGTGAGCT-3'
and
3'-
GACATTCACACCGTTCACGTTATGACTGATGTCACTGACATATGTACTTCGGTAGTTCTGTTTGATGACGTGGTTTGGAGTCTTCAGGATGACTT
CCGTCCAC-5'.

The ligation mixture was used to transform DH5-*alpha* E. coli. and miniprep plasmid DNA obtained from ampicillin resistant colonies was screened for the presence of an approximately 0.6Kbp fragment released by digestion with AccI. After DNA sequencing was performed to confirm that the construct encoded the desired sequence, it was cut with PvuII and ligated with the 1.6Kbp fragment of pSVL-hCGβ'. The ligation product was transformed into DH5-alpha strain E. coli. and positive clones were selected. Plasmid DNA was prepared by boiling lysis and digested with EcoNI and XhoI. DNA which had the insert in the correct orientation produced fragments approximately 2.6Kbp, 1.7Kbp, 0.5Kbp, 0.25Kbp, and 0.15Kbp. The plasmid DNA was then cotransfected into COS-7 cells (obtained from the American Type Culture Collection) along with pSVL-hCG-alpha, a pSVL-based plasmid encoding the alpha-subunit (10, 21), using a DEAE-dextran procedure (10, 21, 23, 24). Beginning in 1-2 days and for a few days thereafter, the COS-7 cells produced significant amounts of the free subunits and the heterodimer. These were present in the culture media and heterodimer was detected using sandwich immunoassays employing monoclonal antibodies A113 and B105 (10). The concentration of the protein was concentrated by ultrafiltration and monitored for its abilities to bind to LH and FSH receptors by radioligand receptor assays using ¹²⁵I-hCG and ¹²⁵I-hFSH as tracers and rat ovarian corpora lutea and bovine testes as sources of LH and FSH receptors as described (10).

The procedures set out below produce the alpha, beta-heterodimeric polypeptides in a transient fashion. As is well known in the art, vectors other than pSVL can be chosen which will enable the protein to be expressed in a stable fashion in larger quantities. The DNA sequences described above can be excised from pSVL and then subcloned into any other expression vector. Cell types other than COS-7 cells can be used to express the analogs. These include virtually any eucaryotic cell for which an expression vector is known or can be devised and include mammalian cells, insect cells, yeast cells, fungal cells, and plant cells.

The abilities of the analogs to inhibit binding of ¹²⁵I-hCG to rat corpora luteal LH receptors was performed by procedures similar to those described previously. Immature rats were given injections of pregnant mares serum gonadotropin and hCG to induce the formation of corpora lutea. The ovaries were removed from the animals, homogenized, and stored at a concentration of approximately 5mg homogenate tissue in 100 ul buffer (25).

The abilities of the analogs to inhibit binding of ¹²⁵I-hFSH to bovine testes tissue homogenates was performed by procedures similar to those described previously (10).

Figures 1 and 2 depict in graphic format the ability of hCG, hFSH, and the alpha, beta-heterodimeric polypeptide analogs of the present invention to inhibit binding of ¹²⁵I-hCG to rat corpora luteal LH receptors and binding of ¹²⁵I-hFSH to bovine testes tissue homogenates, respectively.

To obtain additional evidence for our observation that the determinants of LH and FSH receptor binding specificity are independent, we prepared a series of analogs of hCG which had a wide range of LH/FSH activities. We started with the analog that had approximately equal LH and FSH binding activities (this analog has been called a variety of names including "G" and "CF101-109"). This anaolg contains hFSH *beta*-subunit residues 95-103 for hCG *beta*-subunit residues 101-109 in an hCG *beta*-subunit analog which is truncated after residue 114. The sequence of this analog is set out below. The residues derived from the hFSH *beta*-subunit are underlined. The leader sequence is omitted.

9 23 26 34 38 57 72

SKEPLRPRCRPINATLAVEKEGCPVCITVNTTICAGYCPTMTRVLQGVLPALPQVVCNYRDVRFESIRLPGC

88 93 100 110

PRGVNPVVSYAVALSCQCALCRRSTTDCTVRGLGPSYCDDPR

To facilitate making analogs in the 94-97 region, we made intermediate analogs MB135 and MB140. These were modified to give the desired analogs using cassette mutagenesis with short synthetic DNA oligos (i.e., 18-mers).

ANALOG MB135: (Converts G¹⁰²/V in hCG *beta*-subunit residues 1-114.) This analog was made after considering the sequences of all mammalian glycoprotein hormones with LH and FSH activities. One of these, equine LH, has the ability to bind to rat LH and FSH receptors. Equine LH is very similar to hCG except in the region between 101-109 (26). One residue which might be critical in this region is the valine at 102. Therefore, we changed the glycine found in hCG at position 102 to valine.

We synthesized oligos 435 and 436 having the following sequences:

435: 5'-GTGGCTCTCAGCTGTCAATGCGCGCTCTGCCGCAGATCTACCACTGACTGCGGGGTCCCTAAGGACCAC-3', and

436: 3'-TGACTGACGCCCCAGGGATTCCTGGTGGGGAACTGGACACTACTGGGGGCGATTCTCGAGCCTAGGCACACC-5'.

These were annealed and filled-in using Taq polymerase to give a product which encodes amino acids homologous to those of hCG *beta*-subunit between 84-114 except at residue 102 and introduces the BssHII restriction site in the DNA as shown:

88 93 100 110
V A L S C Q C A L C R R S T T D C G V P K D H P L T C D D P R *
GTGGCTCTCAGCTGTCAATGCGCGCTCTGCCGCAGATCTACCACTGACTGCGGGGTCCCTAAGGACCACCCCTTGACCTGTCATGACCCCCGCTA
AGAGCTCGGATCCGTGAGG
CACCGAGAGTCGACAGTTACGCGCGAGACGGCGTCTAGATGCTGACTGACGCCCCAGGGATTCCTGGTGGGGAACTGGACACTACTGGGGGCGAT
TCTCGAGCCTAGGCACACC
CAG^CTG G^CGCGC A^GATCT GACNNNN^NNGTC(DrdI)
GAGCT^CGG^ATCC
PvuII BssHII BglII PG^GWCCY(PpuMI)
(SacI-BamHI)
CCTNAGG(MstII)

(In this diagram, the sequences derived from the original oligos are underlined. The locations of restriction sites that were created are also noted.) The reaction product was subjected to endonuclease digestion with PvuII and SacI, purified on an agarose gel, and cloned into the PvuII-SacI sites of pSVL-hCG-*beta*' to give pSVL-MB135. [pSVL-hCG-*beta*' was made by ligating the small XhoI-BamHI fragment from pKBM-hCG-*beta*' into the XhoI-BamHI sites of pSVL (27). The construct was sequenced using standard dideoxy double strand sequencing methods. pSVL-MB135 was then co-transfected with pSVL-hCG-*alpha* (27) into COS-7 cells. We concentrated the media by placing them in a dialysis bag and then surrounding the dialysis bag with Aquacide II (Calbiochem) overnight at 4° C. The concentrated media were dialyzed against phosphate buffered isotonic saline (PBS). The amount of dimer was measured using a sandwich immunoassay procedure as described (28) except that antibody A113 (*alpha*-subunit specific) and radioiodinated antibody B105 (*beta*-subunit specific) were used as the capture and detection reagents, respectively. This analog formed dimer and bound to LH receptors and stimulated cyclic AMP formation. It had low ability to bind to FSH receptors.

ANALOG MB140: This analog was made from pSVL-MB135 and was engineered to have hFSH *beta*-subunit residues between 101-109. In addition, coding sequences for Aspartic acid and Leucine were added and that for Arginine 94 was eliminated. The latter change added a BspMI site. The large fragment of BssHI/BspMI digestion will be referred to as pSVL-MB140*. This fragment lacked coding sequences corresponding to residues 92, 93, 94, 95, and 96 of the hCG *beta*-subunit. Thus, we could add any coding sequence to this region by ligating pSVL-MB140* with any synthetic DNA cassette. The residues which were most studied corresponded to those for residues 94, 95, and 96 of hCG *beta*-subunit.

Oligos 438 and 439 used to make analog MB140 were made and had the sequences:

439: 5'-CTGTCAATGCGCGCTCTGCGACCTGCGAAGTACTACCGACTGCACTGTCCGCGGTCTTGGGCC-3' and

438: 3'-CGTGACAGGCGCCAGAACCCGGGAGAATAACACTACTAGGGGCGATTCTCGAGCCTAGGCACACC-5'.

These were annealed, extended with Taq polymerase, digested with BssHII and SacI, and then cloned into the BssHII and SacI sites of pSVL-MB135 by standard methods to give pSVL-MB140. The resulting vector had the following coding sequence for amino acids 84-114:

88 93 101 111
V A L S C Q C A L C D L R S T T D C T V R G L G P S Y C D D P R
*
GTGGCTCTCAGCTGTCAATGCGCGCTCTGCGACCTGCGAAGTACTACCGACTGCACTGTCCGCGGTCTTGGGCCCTCTTATTGTGATGATCCTAG
ATAAGAGCTCGGATCCGTGAGG
CACCGAGAGTCGACAGTTACGCGCGAGACGCTGGACGCTTCATGATGGCTGACGTGACAGGCGCCAGAACCCGGGAGAATAACACTACTAGGGGC
GATTCTCGAGCCTAGGCACACC
CAG^CTG G^CGCGC ACCTGC(BspMI) GACNNNN^NNGTC(DrdI) GGGCCC(ApaI)
GAGCT^CGG^ATCC
PvuII BssHII AGTACT(ScaI) CCGC^GG(SstII)
(SacI-BamHI)

Note, as before, the sequence derived from the primers is underlined and several restriction sites are identified. pSVL-MB140 was expressed in COS-7 cells along with pSVL-hCG-*alpha.* This analog had limited ability to form dimer with the *alpha*-subunit and was nearly inactive in most biological assays. The analog was readily detected in a *beta*-subunit specific sandwich assay employing antibodies B201 and radiolabeled B105 as capture and detection antibodies, respectively (Table 5). This indicated that it probably folded similarly to the *beta*-subunit.

ANALOG MB144: (This changes the sequence DLRST of ANALOG MB140 to DRST).

Synthetic oligos 440 and 441 having the sequences: 
440 5'-CGCGTTGTGTGACAGATC-3' and
441 3'-AACACACTGTCTAGATGA-5'
 were ligated into pSVL-MB140* by standard methods to give pSVL-MB144:

88 93 100
C A L C D R S T T D C
TGCGCGTTGTGTGACAGATCTACTACCGACTGC
ACGCGCAACACACTGTCTAGATGATGGCTGACG
AGATCT
BglII

A BglII site was created and the ScaI and BssHII sites were destroyed. The change in sites was used for screening of the DNA construct during cloning. After its structure had been confirmed by DNA sequencing, pSVL-MB144 was co-transfected into COS-7 cells with pSVL-hCG-*alpha.* Media were concentrated and found to contain *alpha*, *beta*-heterodimer using the sandwich assay. This analog bound to LH and FSH receptors and stimulated cyclic AMP accumulation (Figures 5 and 7). The ratio of LH to FSH activity is described in Figure 4c.

ANALOG MB145: (This changes the sequence DLRST of ANALOG MB140 to NRST).

Synthetic oligos 442 and 443 having the sequences: 
442 5′-CGCGTTGTGTAACAGATC-3′ and
443 3′-AACACATTGTCTAGATGA-5′
 were ligated into pSVL-MB140* to give pSVL-MB145:

93 100
C A L C N R S T T D C
TGCGCGTTGTGTAACAGATCTACTACCGACTGC
ACGCGCAACACATTGTCTAGATGATGGCTGACG
AGATCT
BglII

A BglII site was created and the ScaI and BssHII sites of pSVL-MB140* were destroyed. The change in sites was used for screening of the DNA construct during cloning. After its structure had been confirmed by DNA sequencing, pSVL-MB145 was co-transfected into COS-7 cells with pSVL-hCG-*alpha*. Media were concentrated and found to contain *alpha*, *beta*-heterodimer using the sandwich assay. This analog bound to LH and FSH receptors and stimulated cyclic AMP accumulation (Figures 5 and 7). The LH:FSH activity ratio is described in Figure 4c.

ANALOG MB146: (This changes the sequence DLRST of ANALOG MB140 to RSST).

Synthetic oligos 444 and 445 having the sequences: 
444 5'-CGCGTTGTGTAGATCTTC-3' and
445 3'-AACACATCTAGAAGATGA-5'
 were ligated into pSVL-MB140* by standard methods to give pSVL-MB146:

88 93 100
C A L C R S S T T D C
TGCGCGTTGTGTAGATCTTCTACTACCGACTGC
ACGCGCAACACATCTAGAAGATGATGGCTGACG
AGATCT
BglII

A BglII site was created and the ScaI and BssHII sites were destroyed. The change in sites was used for screening of the DNA construct during cloning. After its structure had been confirmed by DNA sequencing, pSVL-MB146 was co-transfected into COS-7 cells with pSVL-hCG-*alpha.* Media were concentrated and found to contain *alpha*, *beta*-heterodimer using the sandwich assay. This analog bound to LH and FSH receptors and stimulated cyclic AMP accumulation (Figures 5 and 7). The ratio of LH to FSH activity is described in Figure 4c.

ANALOG MB147: (This changes the sequence DLRST of ANALOG MB140 to RSDT).

Synthetic oligos 447 and 448 having the sequences: 
447 5′-CGCGTTGTGTAGATCTGA-3′ and
448 3′-AACACATCTAGACTATGA-5′
 were ligated into pSVL-MB140* by standard methods to give pSVL-MB147:

93 100
C A L C R S D T T D C
TGCGCGTTGTGTAGATCTGATACTACCGACTGC
ACGCGCAACACATCTAGACTATGATGGCTGACG
AGATCT
BglII

A BglII site was created and the ScaI and BssHII sites were destroyed. The change in sites was used for screening of the DNA construct during cloning. After its structure had been confirmed by DNA sequencing, pSVL-MB147 was co-transfected into COS-7 cells with pSVL-hCG-*alpha.* Media were concentrated and found to contain *alpha*, *beta*-heterodimer using the sandwich assay. This analog bound to LH and FSH receptors and stimulated cyclic AMP accumulation (Figures 5 and 7). The ratio of LH to FSH activity is described in Figure 4c.

ANALOG MB148: (This changes the sequence DLRST of ANALOG MB140 to RSNT).

Synthetic oligos 449 and 450 having the sequences: 
449 5′-CGCGTTGTGTAGATCTAA-3′ and
450 3′-AACACATCTAGATTATGA-5′
 were ligated into pSVL-MB140* by standard methods to give pSVL-MB148:

93 100
C A L C R S N T T D C
TGCGCGTTGTGTAGATCTAATACTACCGACTGC
ACGCGCAACACATCTAGATTATGATGGCTGACG
AGATCT
BglII

A BglII site was created and the ScaI and BssHII sites were destroyed. The change in sites was used for screening of the DNA construct during cloning. After its structure had been confirmed by DNA sequencing, pSVL-MB148 was co-transfected into COS-7 cells with pSVL-hCG-*alpha*. Media were concentrated and found to contain *alpha*, *beta*-heterodimer using the sandwich assay. This analog bound to LH and FSH receptors and stimulated cyclic AMP accumulation (Figures 5 and 7). The ratio of LH to FSH activity is described in Figure 4c.

ANALOG MB149: (This changes the sequence DLRST of ANALOG MB140 to QRST).

Synthetic oligos 451 and 452 having the sequences: 
451 5′-CGCGTTGTGTCAGAGATC-3′ and
452 3′-AACACAGTCTCTAGATGA-5′
 were ligated into pSVL-MB140* by standard methods to give pSVL-MB149:

93 100
C A L C Q R S T T D C
TGCGCGTTGTGTCAGAGATCTACTACCGACTGC
ACGCGCAACACAGTCTCTAGATGATGGCTGACG
AGATCT
BglII

A BglII site was created and the ScaI and BssHII sites were destroyed. The change in sites was used for screening of the DNA construct during cloning. After its structure had been confirmed by DNA sequencing, pSVL-MB149 was co-transfected into COS-7 cells with pSVL-hCG-*alpha*. Media were concentrated and found to contain *alpha*, *beta*-heterodimer using the sandwich assay. This analog bound to LH and FSH receptors and stimulated cyclic AMP accumulation (Figures 5 and 7). The ratio of LH to FSH activity is described in Figure 4c.

ANALOG MB150: (This changes the sequence DLRST of ANALOG MB140 to RQST).

Synthetic oligos 514 and 515 having the sequences: 
514 5′-CGCGCTGTGTCGACAGAG-3′ and
515 3′-GACACAGCTGTCTCATGA-5′
 were ligated into pSVL-MB140* by standard methods to give pSVL-MB150:

93 100
C A L C R Q S T T D C
TGCGCGCTGTGTCGACAGAGTACTACCGACTGC
ACGCGCGACACAGCTGTCTCATGATGGCTGACG
G^CGCGC G^TCGAC A^GTACT
BssHIII SalI ScaI

Note, the ScaI and BssHII sites were not destroyed and a SalI site was added for screening. After its structure had been confirmed by DNA sequencing, pSVL-MB150 was co-transfected into COS-7 cells with pSVL-hCG-*alpha.* Media were concentrated and found to contain *alpha*, *beta*-heterodimer using the sandwich assay. This analog bound to LH and FSH receptors and stimulated cyclic AMP accumulation (Figures 5 and 7). The LH:FSH activity ratio is described in Figure 4c.

ANALOG MB151: (This changes the sequence DLRST of ANALOG MB140 to DSRT).

Synthetic oligos 455 and 456 having the sequences: 
455 5′-CGCGTTGTGTGATTCTCG-3′ and
456 3′-AACACACTAAGAGCATGA-5′
 were ligated into pSVL-MB140* by standard methods to give pSVL-MB151:

93 100
C A L C D S R T T D C
TGCGCGTTGTGTGATTCTCGTACTACCGACTGC
ACGCGCAACACACTAAGAGCATGATGGCTGACG

The ScaI and BssHII sites were destroyed and this information was used for screening. After its structure had been confirmed by DNA sequencing, pSVL-MB151 was co-transfected into COS-7 cells with pSVL-hCG-*alpha.* Media were concentrated and found to contain *alpha*, *beta*-heterodimer using the sandwich assay. This analog bound to LH and FSH receptors and stimulated cyclic AMP accumulation (Figures 5 and 7). The ratio of LH to FSH activity is described in Figure 4c.

ANALOG MB152: (This changes the sequence DLRST of ANALOG MB140 to DSAT).

Synthetic oligos 457 and 458 having the sequences: 
457 5′-CGCGTTGTGTGATTCTGC-3′ and
458 3′-AACACACTAAGACGATGA-5′
 were ligated into pSVL-MB140* by standard methods to give pSVL-MB152:

93 100
C A L C D S A T T D C
TGCGCGTTGTGTGATTCTGCTACTACCGACTGC
ACGCGCAACACACTAAGACGATGATGGCTGACG

The ScaI and BssHII sites were destroyed and this information was used for screening. After its structure had been confirmed by DNA sequencing, pSVL-MB152 was co-transfected into COS-7 cells with pSVL-hCG-*alpha.* Media were concentrated and found to contain *alpha*, *beta*-heterodimer using the sandwich assay. This analog bound to LH and FSH receptors and stimulated cyclic AMP accumulation (Figures 5 and 7). The ratio of LH to FSH activity is described in Figure 4c.

ANALOG MB153: (This changes the sequence DLRST of ANALOG MB140 to DRDT).

Synthetic oligos 459 and 460 having the sequences: 
459 5'-CGCGTTGTGTGATAGAGA-3' and
460 3'-AACACACTATCTCTATGA-5'
 were ligated into pSVL-MB140* by standard methods to give pSVL-MB153:

93 100
C A L C D R D T T D C
TGCGCGTTGTGTGATAGAGATACTACCGACTGC
ACGCGCAACACACTATCTCTATGATGGCTGACG

The ScaI and BssHII sites were destroyed and this information was used for screening. After its structure had been confirmed by DNA sequencing, pSVL-MB153 was co-transfected into COS-7 cells with pSVL-hCG-*alpha.* Media were concentrated and found to contain *alpha*, *beta*-heterodimer using the sandwich assay. This analog bound to LH and FSH receptors and stimulated cyclic AMP accumulation (Figures 5 and 7). The ratio of LH to FSH activity is described in Figure 4c.

ANALOG MB154: (This changes the sequence DLRST of ANALOG MB140 to ASDT).

Synthetic oligos 461 and 462 having the sequences: 
461 5′-CGCGTTGTGTGCTAGCGA-3′ and
462 3′-AACACACGATCGCTATGA-5′
 were ligated into pSVL-MB140* by standard methods to give pSVL-MB154:

93 100
C A L C A S D T T D C
TGCGCGTTGTGTGCTAGCGATACTACCGACTGC
ACGCGCAACACACGATCGCTATGATGGCTGACG
G^CTAGC
NheI

The ScaI and BssHII sites were destroyed and a NheI site was created. This information was used for screening. After its structure had been confirmed by DNA sequencing, pSVL-MB154 was co-transfected into COS-7 cells with pSVL-hCG-*alpha*. Media were concentrated and found to contain *alpha*, *beta*-heterodimer using the sandwich assay. This analog bound to LH and FSH receptors and stimulated cyclic AMP accumulation (Figures 5 and 7). The ratio of LH to FSH activity is described in Figure 4c.

ANALOG MB155: (This changes the sequence DLRST of ANALOG MB140 to QIST).

Synthetic oligos 463 and 464 having the sequences: 
463 5′-CGCGTTGTGTCAGATCTC-3′ and
464 3′-AACACAGTCTAGAGATGA-5′
 were ligated into pSVL-MB140* by standard methods to give pSVL-MB155:

93 100
C A L C Q I S T T D C
TGCGCGTTGTGTCAGATCTCTACTACCGACTGC
ACGCGCAACACAGTCTAGAGATGATGGCTGACG
A^GATCT
BglII

A BglII site was created and the ScaI and BssHII sites were destroyed. The change in sites was used for screening of the DNA construct during cloning. After its structure had been confirmed by DNA sequencing, pSVL-MB155 was co-transfected into COS-7 cells with pSVL-hCG-*alpha.* Media were concentrated and found to contain *alpha*, *beta*-heterodimer using the sandwich assay. This analog bound to LH and FSH receptors and stimulated cyclic AMP accumulation (Figures 5 and 7). The ratio of LH to FSH activity is described in Figure 4c.

ANALOG MB192: (This changes the sequence DLRST of ANALOG MB140 to RDST).

Synthetic oligos 516 and 517 having the sequences: 
516 5′-CGCGCTGTGTCGCGATAG-3′ and
517 3′-GACACAGCGCTATCATGA-5′
 were ligated into pSVL-MB140* by standard methods to give pSVL-MB192:

93 100
C A L C R D S T T D C
TGCGCGCTGTGTCGCGATAGTACTACCGACTGC
ACGCGCGACACAGCGCTATCATGATGGCTGACG
G^CGCGC TCGCGA A^GTACT
BssHIII NruI ScaI

An NruI site was created and used for screening of the DNA construct during cloning. After its structure had been confirmed by DNA sequencing, pSVL-MB192 was co-transfected into COS-7 cells with pSVL-hCG-*alpha*. Media were concentrated and found to contain *alpha*, *beta*-heterodimer using the sandwich assay. This analog bound to LH and FSH receptors and stimulated cyclic AMP accumulation (Figures 5 and 7). The ratio of LH to FSH activity is described in Figure 4c.

ANALOG MB201: (This changes the sequence DLRST of ANALOG MB140 to RNST).

Synthetic oligos 518 and 519 having the sequences: 
518 5′-CGCGCTGTGTCGGAATTC-3′ and
519 3′-GACACAGCCTTAAGATGA-5′
 were ligated into pSVL-MB140* by standard methods to give pSVL-MB201:

93                    100
C  A  L  C  R  N  S  T  T  D  C
TGCGCGCTGTGTCGGAATTCTACTACCGACTGC
ACGCGCGACACAGCCTTAAGATGATGGCTGACG
G^CGCGC          G^AATTC
BssHIII           EcoRI

An EcoRI site was created and used for screening of the DNA construct during cloning. After its structure had been confirmed by DNA sequencing, pSVL-MB201 was co-transfected into COS-7 cells with pSVL-hCG-*alpha.* Media were concentrated and found to contain *alpha*, *beta*-heterodimer using the sandwich assay. This analog bound to LH and FSH receptors and stimulated cyclic AMP accumulation (Figures 5 and 7). The ratio of LH to FSH activity is described in Figure 4c.

ANALOG MB194: (This changes the sequence DLRST of ANALOG MB140 to RSRT).

Synthetic oligos 520 and 521 having the sequences: 
520 ′ 5′-CGCGCTGTGTAGATCTCG-3′ and
521 3′-GACACATCTAGAGCATGA-5′
 were ligated into pSVL-MB140* by standard methods to give pSVL-MB194:

93 100
C A L C R S R T T D C
TGCGCGCTGTGTAGATCTCGTACTACCGACTGC
ACGCGCGACACATCTAGAGCATGATGGCTGACG
G^CGCGC A^GATCT
BssHIII BglII

A BglII site was created and used for screening of the DNA construct during cloning. After its structure had been confirmed by DNA sequencing, pSVL-MB194 was co-transfected into COS-7 cells with pSVL-hCG-*alpha.* Media were concentrated and found to contain *alpha*, *beta*-heterodimer using the sandwich assay. This analog bound to LH and FSH receptors and stimulated cyclic AMP accumulation (Figures 5 and 7). The ratio of LH to FSH activity is described in Figure 4c.

ANALOG MB202: (This changes the sequence DLRST of ANALOG MB140 to DSDT).

Synthetic oligos 522 and 523 having the sequences: 
522 5′-CGCGCTGTGTGATTCTGA-3′ and
523 3′-GACACACTAAGACTATGA-5′
 were ligated into pSVL-MB140* by standard methods to give pSVL-MB202:

93 100
C A L C D S D T T D C
TGCGCGCTGTGTGATTCTGATACTACCGACTGC
ACGCGCGACACACTAAGACTATGATGGCTGACG
G^CGCGC G^ANTC
BssHIII HinfI

A HinfI site was created and used for screening of the DNA construct during cloning. After its structure had been confirmed by DNA sequencing, pSVL-MB202 was co-transfected into COS-7 cells with pSVL-hCG-*alpha.* Media were concentrated and found to contain *alpha*, *beta*-heterodimer using the sandwich assay. This analog bound to LH and FSH receptors and stimulated cyclic AMP accumulation (Figures 5 and 7). The ratio of LH to FSH activity is described in Figure 4c.

ANALOG MB278: (This changes the sequence DLRST of ANALOG MB140 to RRRT).

Synthetic oligos 628 and 629 having the sequences: 
628 5′-CGCGCTCTGTCGACGGCG-3′ and
629 3′-GAGACAGCTGCCGCATGA-5′
 were ligated into pSVL-MB140* by standard methods to give pSVL-MB278:

93 100
C A L C R R R T T D C
TGCGCGCTCTGTCGACGGCGTACTACCGACTGC
ACGCGCGAGACAGCTGCCGCATGATGGCTGACG
G^CGCGC G^TCGAC
BssHII SalI

A SalI site was created and used for screening of the DNA construct during cloning. After its structure had been confirmed by DNA sequencing, pSVL-MB278 was co-transfected into COS-7 cells with pSVL-hCG-*alpha*. Media were concentrated and found to contain *alpha*, *beta*-heterodimer using the sandwich assay. This analog bound to LH and FSH receptors (Figures 4a and 4b). The ratio of LH to FSH activity is described in Figure 4c.

ANALOG MB279: (This changes the sequence DLRST of ANALOG MB140 to DDDT).

Synthetic oligos 630 and 631 having the sequences: 
630 5'-CGCGTTATGCGATGACGA-3' and
631 3'-AATACGCTACTGC[?]ATGA-5'
 were ligated into pSVL-MB140* by standard methods to give pSVL-MB279:

93 100
C A L C D D D T T D C
TGCGCGTTATGCGATGACGATACTACCGACTGC
ACGCGCAATACGCTACTGCTATGATGGCTGACG

The BssHII site was destroyed and this observation was used for screening the DNA construct during cloning. After its structure had been confirmed by DNA sequencing, pSVL-MB279 was co-transfected into COS-7 cells with pSVL-hCG-*alpha*. Media were concentrated and found to contain *alpha*, *beta*-heterodimer using the sandwich assay. This analog bound to FSH receptors but had low affinity for LH receptors (Figures 4a and 4b). The ratio of LH to FSH activity is described in Figure 4c.

ANALOG PRM3: (This is a full-length hCG *beta*-subunit with hFSH *beta*-subunit residues 88-100 substituted for hCG *beta*-subunit residues 94-106.) This analog was made using oligonucleotides 363, 364, 365, 368, 508, and 510. The sequences of these oligonucleotides are illustrated below:

363: 5′-AGCTGTCCTGGAGCTAGGAATCT-3′
364: 5′-CTAGCCTAGAAGCTCTGACTGTC-3′
365:5′-AGCTGTCCTGGAGCTAGGAATCTCTGTACGGAAGTGTTACTTCTGCTCT-
3′
368:5′-CTAGCCTAGAAGCTCTGACTGTCCTAGTTGTGGTTTGTCCAAACTCATC-
3′
508: 5′-ACTGTCCGCGGCTTGGGTCCCTTGACCTGTGATGACCCCCGCTTC-3′
510: 5′-GGGACCCAAGCCGCGGACAGTACAGTCAGTACTGTCGCTGTCGCAGAG-
3′

Oligos 365 and 510 were used in a PCR reaction using conditions similar to those described (29) with CF94-117 (27) as a primer. Oligos 508 and 368 were used in a second PCR reaction using pSVL-hCG-*beta*' as a primer. The products of the first and second reactions were mixed and a third PCR reaction performed using oligos 363 and 364. The resulting DNA was then digested with XbaI and BamHI endonucleases, purified on agarose, and ligated into the XbaI/BamHI sites of pSVL. The sequence of the DNA between the XbaI and BamHI sites was confirmed by double stranded dideoxy sequencing. Note also that this introduced an SstII site which was used in further cloning.

93 100 110
L C D S D S T D C T V R G L G P L T C D D P R F
CTCTGCGACAGCGACAGTACTGACTGTACTGTCCGCGGCTTGGGTCCCTTGACCTGTGATGACCCCCGCTTC
GAGACGCTGTCGCTGTCATGACTGACATGACAGGCGCCGAACCCAGGGAACTGGACACTACTGGGGGCGAAG
AGT^ACT CCGC^GG
ScaI SstII

The portions of the molecule between residues 1-93 and 107-145 were identical in sequence to those of the hCG *beta*-subunit. This analog was co-transfected into COS-7 cells along with pSVL-hCG-*alpha* and the media were collected on the third day after transfection (27). The media were concentrated and analyzed by a sandwich immunoassay using antibodies A113 and radiolabeled B105. This analog combined with the *alpha*-subunit. It had relatively low ability to bind to either LH or FSH receptors (Table 6).

ANALOG PRM8: This is an analog identical to MB202 except that Thr97 of MB202 was replaced by Ser. PRM8 was made by replacing the XhoI/SstII fragment of MB202 with that of PRM3. It was made to learn if the higher than expected amount of LH activity found in MB202 relative to CF94-117 (Table 6) was due to the Thr at residue 97. This residue is Ser in hFSH *beta*-subunit. This analog was co-transfected into COS-7 cells along with pSVL-hCG-*alpha* and the media were collected for assay on the third day after transfection (27). The media were concentrated and analyzed by a sandwich immunoassay using antibodies A113 and radiolabeled B105. This analog combined with the *alpha*-subunit. It had good ability to bind to FSH receptors. It bound to LH receptors better than CF94-117 indicating that Thr97 was not responsible for discriminating LH and FSH receptors.

ANALOGS PRM1 and PRM2: (These are full-length hCG *beta*-subunits. PRM1 contains hFSH *beta*-subunit residues 88-108 substituted for hCG *beta*-subunit residues 94-114. PRM2 is identical to PRM1 except that Tyr109 is replaced with a Phe. PRM2 was made to test the role of the tyrosine at residue 109.) These analogs were prepared using oligonucleotides 596 and 368. The sequence of 368 is listed above and that for 596 is shown below:

596: 5′-TGCACTGTCCGCGGTCTTGGCCCAAGCT(A/T)TTGCAGCTTCGGCGAATTCCAGGACTCCTCTTCCTCAAAGGCC-3′

The products of the PCR reaction were digested with SstII and BamHI and used to replace the small SstII/BamHI fragment of pSVL-PRM3. Due to the use of two bases (i.e., A or T) at one position in this oligonucleotide, we anticipated that there would be two analogs which could be formed. These two analogs differed by the presence of a HindIII restriction endonuclease digestion site (c.f., diagram below) and this difference enabled us to distinguish the analogs during cloning. Both analogs contained an EcoRI site. The sequences of both analogs were confirmed by double stranded dideoxy methods.

### ANALOG PRM1:

100                                    110                                    120

C  T  V  R  G  L  G  P  S  Y  C  S  F  G  E  F  Q  D  S  S  S  S  K  A

TGCACTGTCCGCGGTCTTGGCCCAAGCTATTGCAGCTTCGGCGAATTCCAGGACTCCTCTTCCTCAAAGGCC

ACGTGACAGGCGCCAGAACCGGGTTCGATAACGTCGAAGCCGCTTAAGGTCCTGAGGAGAAGGAGTTTCCGG

CCGC^GG                                    G^AATTC

SstII                                      EcoRI

### ANALOG PRM2:

100 110 120
C T V R G L G P S F C S F G E F Q D S S S S K A
TGCACTGTCCGCGGTCTTGGCCCAAGCTTTTGCAGCTTCGGCGAATTCCAGGACTCCTCTTCCTCAAAGGCC
ACGTGACAGGCGCCAGAACCGGGTTCGAAAACGTCGAAGCCGCTTAAGGTCCTGAGGAGAAGGAGTTTCCGG
CCGC^GG A^AGCTT G^AATTC
SstII HindIII EcoRI

pSVL-PRM1 was co-transfected into COS-7 cells along with pSVL-hCG-*alpha* and the media were collected on the third day after transfection (27). The media were concentrated and analyzed by a sandwich immunoassay using antibodies A113 and radiolabeled B105. This analog combined with the *alpha*-subunit. It had high ability to bind to FSH receptors and relatively low ability to bind to LH receptors (Table 6).

pSVL-PRM2 was co-transfected into COS-7 cells along with pSVL-hCG-*alpha* and the media were collected on the third day after transfection (27). The media were concentrated and analyzed by a sandwich immunoassay using antibodies A113 and radiolabeled B105. This analog combined with the *alpha*-subunit. It had high ability to bind to FSH receptors and relatively low ability to bind to LH receptors (Table 6).

ANALOG PRM10: This is a full-length hCG *beta*-subunit analog in which residues 101-106 are replaced with residues 95-100 of hFSH *beta*-subunit. This analog was made by PCR using oligonucleotides 562 and 365 as PCR primers and MB135 as template. The resulting PCR product was cloned into the XhoI/SstII sites of PRM3. The structure of PRM10 was confirmed by double stranded dideoxy sequencing. 562: 5′-CCCAAGACCGCGGACAGTGCAGTCAGTGGTAGATCTGCG-3′ The sequence of PRM10 in the region 92-107 is:

93 100 107
L C R R S T T D C T V T G L G P
CTCTGCCGCAGATCTACCACTGACTGCACTGTCCGCGGTCTTGGGCCC
GAGACGGCGTCTAGATGGTGACTGACGTGACAGGCGCCAGAACCCGGG
A^GATCT CCGC^GG GGGCC^C
BglII SstII ApaI

ANALOG MB197: This is an analog of CF101-109 with the deletion of Arg95. It was made by ligating the large fragment obtained by digestion of pSVL-MB144 with XbaI/BglII with the small fragment obtained by digestion of pSVL-MB194 using the same enzymes. The resulting analog was only 113 residues in length and its sequence between residues 88 and 99 is illustrated here.

88 93 99
C A L C R S T T D C
TGCGCGCTGTGTAGATCTACTACCGACTGC
ACGCGCGACACATCTAGATGATGGCTGACG
G^CGCGC AGATCT
BssHII BglII

pSVL-MB197 was expressed in COS-7 cells along with pSVL-hCG-*alpha.* This analog had low ability to form dimer with the *alpha*-subunit. The analog was readily detected in a *beta*-subunit specific sandwich assay employing antibodies B201 and radiolabeled B105 as capture and detection antibodies, respectively (Table 5). This indicated that it acquired the overall folding pattern of the *beta*-subunit.

ANALOG MB198: This is an analog of CF101-109 with the insertion of a Gln at residue 94. It was made by ligating the large fragment obtained by digestion of MB194 with XbaI and BglII to the small fragment obtained by similar digestion of MB149 to create pSVL-MB194:

93 101
C A L C Q R S R T T D C
TGCGCGTTGTGTCAGAGATCTCGTACTACCGACTGC
ACGCGCAACACAGTCTCTAGAGCATGATGGCTGACG
AGATCT
BglII

The ScaI and BssHII sites were destroyed and a BglII site was created. This information was used for screening. After its structure had been confirmed by DNA sequencing, pSVL-MB198 was co-transfected into COS-7 cells with pSVL-hCG-*alpha*. The analog was readily detected in a *beta*-subunit specific sandwich assay employing antibodies B201 and radiolabeled B105 as capture and detection antibodies, respectively (Table 5). This indicated that the *beta*-subunit folded.

ANALOG MB284: This is an analog of CF101-109 in which two residues have been deleted from the 93-100 region of the molecule. The analog was made by inserting the following oligonucleotide cassette into the large fragment remaining following digestion of pSVL-MB140 using BssHII and SstII. 
632 5'-CGCGCTCTGTCGACGGACCGACTGCACTGTCCGC-3' and
633 3'-GAGACAGCTGCCTGGCTGACGTGACAGG
 This gave pSVL-MB284:

90 93 98 108 112

C A L C R R T D C T V R G L G P S Y C D D P R *

TGCGCGCTCTGTCGACGGACCGACTGCACTGTCCGCGGTCTTGGGCCCTCTTATTGTGATGATCCTAGATAA

ACGCGCGAGACAGCTGCCTGGCTGACGTGAC[?][?]GCGCCAGAACCCGGGAGAATAACACTACTAGGATCTATT

G^CGCGC G^TCGAC CCGC^GG GGGCC^C

BssHII SalI SstII ApaI

A SalI endonuclease restriction site was created and the ScaI site was destroyed. This information was used for screening. After its structure had been confirmed by DNA sequencing, pSVL-MB284 was co-transfected into COS-7 cells with pSVL-hCG-*alpha*. Media were found to contain small amounts of *alpha*, *beta*-heterodimer using the A113/B105 sandwich assay. The analog was readily detected in a *beta*-subunit specific sandwich assay employing antibodies B201 and radiolabeled B105 as capture and detection antibodies, respectively (Table 5). This indicated that the *beta*-subunit folded.

ANALOG PRM26: The small fragment obtained by XhoI and SstII digestion of PRM10 was ligated to the large fragment obtained by XhoI and SstII digestion of PRM1 to give pSVL-PRM26. The resulting construct encoded residues 1-100 derived from the N-terminus of the hCG *beta*-subunit, residues 101-114 derived from the hFSH *beta*-subunit (i.e., corresponding to hFSH *beta*-subunit residues 95-108), and residues 115-14 5 derived from the C-terminus hCG *beta*-subunit. pSVL-PRM26 was co-transfected into COS-7 cells along with pSVL-hCG-*alpha* and the media were collected on the third day after transfection (27). The media were concentrated and analyzed by a sandwich immunoassay using antibodies A113 and radiolabeled B105. This analog combined with the *alpha*-subunit and the dimer had high LH activity (Table 6).

ANALOG MB261: (Changes residues CRNSTTDCTVR of ANALOG MB201 to CRRSTTDCGGR).

Synthetic oligos 599 and 600 having the sequences: 
599 5′-CGCGCTGTGTAGAAGATCTACTACCGACTGCGGCGGCCGC-3′ and
600 3′-GACACATCTTCTAGATGATGGCTGACGCCGCCGG-5′
 were ligated into the large fragment obtained by BssHII and SstII digestion of pSVL-MB201 by standard methods to give pSVL-MB261:

90          93                    100
 C   A   L   C   R   R   S   T   T   D   C   G   G   R
TGCGCGCTGTGTAGAAGATCTACTACCGACTGCGGCGGCCGC
ACGCGCGACACATCTTCTAGATGATGGCTGACGCCGCCGGCG
 G^CGCGC          A^GATCT
  BssHII           BglII

A BglII site was created and used for screening of the DNA construct during cloning. After its structure had been confirmed by DNA sequencing, pSVL-MB261 was co-transfected into COS-7 cells with pSVL-hCG-*alpha*. Media were concentrated and found to contain *alpha*, *beta*-heterodimer using the A113/B105 sandwich assay. This analog bound LH receptors similar to hCG (Table 5). Since 100ng did not inhibit the binding of ¹²⁵I-hFSH to human FSH receptors, it appears to have reduced activity for FSH receptors.

ANALOG MB272: (Changes residues CGGRGLGPSYC of ANALOG MB261 to CGGRKDGPSYC).

Synthetic oligos 624 and 625 having the sequences: 
624 5′-AAGGACGGCC-3′ and
625 3′-CGTTCCTGC-5′
 were ligated into the large fragment obtained by SstII and ApaI digestion of pSVL-MB201 by standard methods to give pSVL-MB272:

90 93 100 110

C A L C R R S T T D C G G R K D G P S Y C

TGCGCGCTGTGTAGAAGATCTACTACCGACTGCGGCGGCCGCAAGGACGGGCCCTCTTATTGT

ACGCGCGACACATCTTCTAGATGATGGCTGACGCCGCCGGCGTTCCTGCCCGGGAGAATAACA

G^CGCGC A^GATCT G^GGCCC

BssHII BglII ApaI

The SstII site found in pSVL-MB261 was destroyed and this was used for screening of the DNA construct during cloning. After its structure had been confirmed by DNA sequencing, pSVL-MB261 was co-transfected into COS-7 cells with pSVL-hCG-*alpha*. Media were concentrated and found to contain *alpha*, *beta*-heterodimer using the A113/B105 sandwich assay. This analog bound LH receptors similar to hCG (Table 5). Since 100ng did not inhibit the binding of ¹²⁵I-hFSH to human FSH receptors, it appears to have reduced activity for FSH receptors.

ANALOG MB275: (Changes residues CGGRGLGPSYC of ANALOG MB201 to CGGRKDGPSYC).

Oligos 624 and 625 were ligated into the large fragment obtained by SstII and ApaI digestion of pSVL-MB201 by standard methods to give pSVL-MB275:

90 93 100 110

C A L C R N S T T D C T V R K D G P S Y C

TGCGCGCTGTGTCGGAATTCTACTACCGACTGCACTGTCCGCAAGGACGGGCCCTCTTATTGT

ACGCGCGACACAGCCTTAAGATGATGGCTGACGTGACAGGCGTTCCTGCCCGGGAGAATAACA

G^CGCGC G^AATTC G^GGCCC

BssHII EcoRI ApaI

The SstII site found in pSVL-MB201 was destroyed and this was used for screening of the DNA construct during cloning. After its structure had been confirmed by DNA sequencing, pSVL-MB261 was co-transfected into COS-7 cells with pSVL-hCG-*alpha*. Media were concentrated and found to contain *alpha*, *beta*-heterodimer using the A113/B105 sandwich assay. This analog bound LH receptors similar to hCG (Table 5). Since 100ng did not inhibit the binding of ¹²⁵I-hFSH to human FSH receptors, it appears to have reduced activity for FSH receptors.

ANALOG MB283: (Changes residues CVTRGLGPSYC of CF101-109 to CVTRGLGPLT).

Synthetic oligos 626 and 627 having the sequences: 
627:          5′-CTTAACTTGTGATGATCCTAGATAAG-3′     and
626:      3′-CCGGGAATTGAACACTACTAGGATCTATTCCTAG-5′
 were ligated with the large fragment of CF101-109 remaining following ApaI and BamHI digestion to give pSVL-MB283:

100                    110

C T V R G L G P L T C D D P R *

TGCACCGTGAGAGGCCTCGGCCCTTAACTTGTGATGATCCTAGATAAGGATCC

ACGTGGCACTCTCCGCACCCGGGAATTGAACACTACTAGGATCTATTCCTAGG

AGG^CCT                    G^GATCC

StuI                    BamHI

The SstI site found in CF101-109 was destroyed and the loss of the site was used for screening of the DNA construct during cloning. After its structure had been confirmed by DNA sequencing, pSVL-MB261 was co-transfected into COS-7 cells with pSVL-hCG-*alpha*. Media were concentrated and found to contain *alpha*, *beta*-heterodimer using the A113/B105 sandwich assay. This analog bound LH receptors similar to hCG (Table 5). Since 100ng did not inhibit the binding of ¹²⁵I-hFSH to human FSH receptors, it appears to have reduced activity for FSH receptors.

### ANALYSES

Quantification of analogs: The analogs were measured in sandwich immunoassays (28) using antibody A113 as a capture reagent and radiolabeled antibody B105 as a detection reagent. hCG was used as the standard. A113 binds to the *alpha*-subunit and B105 binds to the *beta*-subunit. In some cases, the analogs were also quantified using a sandwich assay employing antibodies B109 or B107 as capture reagents and radiolabeled antibody B105 as a detection reagent. Antibodies B105, B107, and B109 bind to residues in the *beta*-subunit not changed in the analogs described here (30). For those analogs which had low activity in the A113/B105 sandwich assay, we measured the free *beta*-subunit to learn if it were being expressed. To do this we substituted antibody B201 for A113 and performed the remainder of the assay as described (28). B201 binds to an epitope on the *beta*-subunit much better than to hCG. To be certain that failure to obtain *alpha*, *beta*-heterodimers was not related to lack of *alpha*-subunit, we also measured the free *alpha*-subunit using antibodies A501 and radiolabeled A202 in place of A113 and radiolabeled B105. A501 binds free *alpha*-subunit much better than hCG.

FSH receptor binding: Binding of analogs to FSH receptors was measured using a CHO cell line that had been transfected with a cDNA encoding the human FSH receptor. This cell line was obtained from AAT, Randolph, MA and was grown in suspension culture in *alpha*MEM containing 10% fetal bovine serum. On the day of the experiment cells were harvested, washed in phosphate buffered saline, and counted. Aliquots of 200,000 to 1,000,000 cells were incubated with ¹²⁵I-hFSH and FSH standards or analogs to be tested for 1 hour at 37½C in Krebs-Hepes buffer (28). The cells were collected by sedimentation at 1000xg for 10-15 min and the supernate containing the non-bound hormone was aspirated. The amount of radioactivity remaining in the cell pellets was determined in a gamma counter.

LH receptor binding: Binding of analogs to LH receptors was determined using a CHO cell line that had been transfected with a cDNA encoding the rat LH receptor (29) or with a cell line that had been transfected with the human LH receptor (31). The procedure used was identical to that described for FSH receptor binding except that ¹²⁵I-hCG and hCG were substituted for ¹²⁵I-hFSH and hFSH.

Cyclic AMP assays: CHO cells expressing rat LH receptors, human LH receptors, or human FSH receptors were incubated with analogs and hCG or hFSH for 10-15 minutes and then placed in a boiling water bath for 1 min. The cyclic AMP was measured using a cyclic AMP immunoassay (32).

### ACTIVITIES OF ANALOGS

Interaction of analogs CF101-109 and CF94-117 with human LH and FSH receptors (Figure 3): CF101-109 and CF94-117 are analogs of both hCG and hFSH. CF94-117 has been shown to bind FSH receptors (27) and here we illustrate that CF101-109 binds both LH and FSH receptors. Previous studies employed rat LH and FSH receptors and bovine FSH receptors (27). We expected that analogs of the human hormones which bound rat receptors would also bind to human receptors and initiate signal transduction. To confirm this prediction, we tested the abilities of CF94-117 (27) and CF101-109 to bind to human LH and FSH receptors stably expressed in Chinese hamster ovary CHO cells. Both CF94-117 and CF101-109 inhibited binding of ¹²⁵I-hFSH to human FSH receptors (Figure 3). In addition, CF101-109 inhibited the binding of ¹²⁵I-hCG to human LH receptors at about the same potency as hCG. CF101-109 and CF94-117 induced cyclic AMP accumulation in CHO cells expressing human FSH receptors, albeit with slightly lower potency than hFSH. It is not possible to provide exact potency estimates since the amounts of analogs used in these studies were determined against an hCG standard in sandwich immunoassays. As illustrated in Table 4, some antibody combinations give quantitatively different results than others. CF101-109 induced rounding of Y-1 cells transfected with genes that stably express LH receptors (i.e., Y-1L cells) or FSH receptors (i.e., Y-1F cells). Rounding of Y-1 cells has long been correlated with responses to hormones such as ACTH for which the cells have ACTH receptors (33). Y-1F cells rounded in response to hFSH but not hCG whereas Y-1L cells rounded in response to hCG but not hFSH. This suggests that rounding is an index of response to any hormone which can induce cyclic AMP formation in cells derived from the original Y-1 line. Since both Y-1F and Y-1L cells respond to CF101-109, this analog must be functionally interacting with FSH and LH receptors in these cells. In addition, the Y-1 cells transfected with FSH receptors made progesterone in response to CF101-109 and hFSH but not to hCG (Figure 4). This further suggests that CF101-109 can elicit a physiological response such as steroid production.

Abilities of analogs of CF101-109 to combine with *alpha*-subunit. To determine if analogs of CF101-109 would combine with the *alpha*-subunit to form a heterodimer, we subjected culture media from transfected cells to sandwich assays that detected *alpha*, *beta* heterodimers. All results were quantified using an hCG standard. Most assays employed A113 (i.e., an antibody specific for the *alpha*-subunit) and B105 (i.e., an antibody specific for the *beta*-subunit). Although B105 does not bind to hFSH, it has been shown to bind to CF94-117 (27). This is an analog in which all residues between 94 and 117 are derived from hFSH *beta*-subunit. Therefore, B105 binds a part of the *beta*-subunit outside the region that was modified in the studies described here. In some studies, we also employed antibodies B107 or B109 and B105. B107 and B109 have also been shown to bind to portions of the *beta*-subunit outside the region modified in these studies (30). Although the antibodies used in these assays were chosen on the basis of the fact that they recognized different regions of the hormone than was modified in these studies, slight changes in the conformations of the *alpha*- and/or *beta*-subunits caused by the mutations may have influenced binding of the analogs to the antibodies (B107 and B110 which are known to be sensitive to conformation of the *beta*-subunit which occurs during formation of the *alpha*, *beta*-heterodimer (30)). We observed differences in the absolute amounts of analogs depending on the sandwich immunoassays which were employed. As a rule, sandwich assays using B109 and B105 gave lower estimates than those assays which employed other antibody combinations (Table 4). None the less, differences in the amounts of material present in the media were not correlated with the activities of the analogs in either LH receptor or FSH receptor assays. It should also be noted that the same batches of analogs were used in FSH and LH receptor assays. Thus, estimates in the amounts of analog present in culture medium do not enter into the calculation of the ratio of LH to FSH activity. Except as noted in Table 4, the data illustrated in all tables and figures were obtained using the A113/B105* sandwich assay.

The results of sandwich immunoassays indicated that all the analogs we made except for MB140, MB197, MB198, and MB284 formed *alpha*, *beta* dimers when co-expressed with the human *alpha*-subunit. Cells expressing the human *alpha*-subunit and MB140, MB197, MB198, or MB284 *beta*-subunits did not produce much *alpha*, *beta*-heterodimer. These were the only analogs in this series which had a different number of residues in the region between Cys93 and Cys100 than is found in all naturally occurring hormone *beta*-subunits (34). Every analog we prepared which contained 6 amino acid residues between Cys93 and Cys100 combined with the *alpha*-subunit and was detected in A113/B105* sandwich immunoassays. These included analogs which contain a glycosylation signal at residue 94 (i.e., MB145), residue 95 (i.e., MB201), or residue 96 (i.e., MB148) as well as analogs with three positive charges (i.e., MB278) or four negative charges (i.e., MB279) between Cys93 and Cys100. Thus, we anticipate that mutations in this region will be tolerated as long as they do not change the size of the Cys93-Cys100 loop.

### EFFECTS OF MUTATIONS IN RESIDUES 94-97 OF CF101-109 ON LH AND FSH ACTIVITY

Results of LH Binding Assays (Figures 3, 4a, and 4c). In the parent application, we noted that CF101-109 bound to rat LH receptors and bovine FSH receptors. As illustrated in Figure 3, CF101-109 binds to human LH receptors and human FSH receptors. In the studies reported here we have used the rat LH receptor and human LH and FSH receptors to test the activities of the CF101-109 analogs. Modification of residues in the region of the molecule between 94-97 altered the affinities of CF101-109 analogs for LH receptors more than it altered their affinities for FSH receptors (Figure 3). The charge of the amino acids in this region was correlated with LH activity (Figures 4a and 4c). Binding to LH receptors was enhanced when at least one positive charge or no negative charges were present in the 94-97 region. Thus, while analogs MB154 (ASDT), MB152 (DSAT), MB202 (DSDT), and MB279 (DDDT) competed well with ¹²⁵I-hFSH for binding to FSH receptors, they had the lowest ability to compete with ¹²⁵I-hCG for binding to LH receptors. Inclusion of a positive charge or replacement of a negative charge with one positive charge resulted in much better binding to LH receptors without substantially altering binding to FSH receptors. Thus, analogs MB151 (DSRT) and MB147 (RSDT) were more active than MB154 (ASDT), MB152 (DSAT), MB202 (DSDT) or MB279 (DDDT) in the LH receptor assay. Elimination of all negative charges was sufficient to increase the LH activity. Thus MB155 (QIST) was more active than MB154 (ASDT), MB152 (DSAT), MB202 (DSDT) or MB279 (DDDT). As a rule, analogs with no negative charges had the greatest ability to bind to LH receptors. These included MB149 (QRST), MB150 (RQST), MB145 (NRST), MB201 (RNST) and MB194 (RSRT). With some exceptions, notably the lower activity of MB147 (RSDT) relative to that of MB151 (DSRT), the locations of the charged residues had only a small influence on binding to LH receptors. For example, both MB149 (QRST) and MB150 (RQST) were very active even though they have Arg residues at different positions. Likewise, the ratio of LH/FSH activity of MB151 (DSRT) was only slightly less than that of MB147 (RSDT). While most position effects were relatively minor, the residue in which a negative charge had the greatest influence on LH receptor binding appeared to be number 96. These results suggest that analogs having high FSH specificity can be prepared by substituting negative charges in the region of the molecule between Cys93 and Cys100. Use of positive charges in this region is expected to lead to enhanced LH receptor interactions.

Results of FSH Binding Assays (Figures 3 and 4b). In general, substitutions in the region between 94 and 97 had a much lower effect in the FSH binding assays than in the LH binding assays. Most analogs were more than 20% as active as FSH in this assay. The major exception to this statement was analog MB153 (DRDT). This analog was more active in LH receptor binding assays than in FSH receptor binding assays even though it had negative charges at residues 94 and 96.

Results of LH-receptor induced cyclic AMP formation assays (Figure 7). Most analogs which bound to LH receptors were capable of stimulating cyclic AMP formation. Their potency relative to hCG was similar to their potency relative to hCG in receptor binding assays.

Results of FSH-receptor induced cyclic AMP formation assays (Figure 7). Most analogs which bound to FSH receptors were capable of stimulating cyclic AMP formation. Their potency in this assay was only approximately 10% of that of their expected potency based on their abilities to bind to FSH receptors. CF94-117 was also somewhat less capable of eliciting cyclic AMP accumulation in CHO cells bearing FSH receptors. This observation is surprising because CF94-117 was nearly equally effective as FSH in inducing steroidogenesis in cultured granulosa cells (27), a response presumed to be mediated by cyclic AMP (35). This, in the case of FSH receptors, we anticipate that the ability of all analogs to stimulate granulosa cell steroidogenesis will be better than their abilities to stimulate cyclic AMP accumulation in cells that stably express the FSH receptor.

### EFFECTS OF MUTATIONS IN RESIDUES 101-114 OF CF101-109 ON LH AND FSH ACTIVITY

The region of the *beta*-subunit between residues 108 and the C-terminus was found to influence the specificity of hormone binding. We observed that analog MB202 had at least 10-fold greater ability than CF94-117 to bind to LH receptors (Table 6). The differences between MB202 and CF94-117 include the substitution of Thr for Ser97 and the sequence AspAspProArg for SerPheGlyGluMetLysGlu at the C-terminus. Previous studies indicated that CF94-114 had equal activity as CF94-117 (27) suggesting that the three residues at the very C-terminus of hFSH (i.e., MetLysGlu) did not have a role in binding specificity. To identify a role for Ser97 in binding specificity, we prepared PRM8. This analog differed from MB202 only in the presence of Ser at position 97. PRM8 bound to LH receptors better than CF94-117 suggesting that Ser97 was not important for discriminating LH and FSH receptors. It also suggested that residues between 111 and 114 or 111 and 117 had an important role in suppressing binding of FSH to LH receptors. To examine an influence of the C-terminus, we prepared PRM1 and PRM2. PRM1 is similar in structure to CF94-114 (27) except that it also contains residues 115-145 of the hCG C-terminus. PRM1 had similar abilities as CF94-117 to distinguish LH and FSH receptors. This suggested that FSH residues 111, 112, 114, and/or 114 were needed to reduce LH receptor binding to the level observed in CF94-114 or CF94-117. The influence of residues 111-114 on LH receptor interaction is lower than that of residues between 94-97. By comparing the activities of PRM26 (Table 6), PRM1 (Table 6), and CF94-97, 108-114 (Table 3), it can be seen that substituting hFSH *beta*-subunit sequences 105-108 for residues 111-114 does not appear to substantially alter the ability of the analog to bind to LH receptors when residues 94-97 or residues 101-106 of the analogs are derived from hCG *beta*-subunit. However, CF94-114, CF94-117, PRM1, and PRM2 have low LH activity. This shows that substitution of hFSH *beta*-subunit residues 105-108 for analogs 111-114 can modulate LH receptor activity when residues 94-108 are derived from hFSH *beta*-subunit. The ability of PRM2 to bind to FSH receptors suggests that substitution of Phe for Tyr109 does not alter binding specificity or affinity.

In further support of a role of residues between 101-109 in FSH receptor binding, we observed that PRM3, an analog which differed from PRM1 by the presence of hCG *beta*-subunit sequences in the region between residues 108-114, bound poorly to LH receptors yet had unexpectedly low affinity for FSH receptors. The low binding of PRM3 to LH receptors was caused by the presence of negative charges in the region of the molecule between residues 94 and 97. Since CF101-109 binds FSH receptors well and PRM3 has low affinity for FSH receptors, it appears that residues 108 and 109 contribute to the ability of the heterodimer to recognize LH and FSH receptors. Suppression of LH binding activity in PRM8 and MB202 requires modification of amino acids between residues 111 and 114. The ability of PRM2 to bind to FSH receptors suggests that substitution of Phe for Tyr109 does not alter binding specificity or affinity.

The following are the preferred analogs of the invention:
CF101-109, MB278: These two analogs give high binding to both LH and FSH receptors and are active in inducing a biological response.
PRM1, PRM2: These two analogs have the greatest selectivity to FSH receptors.
MB202: This analog has high FSH activity with small amounts of LH activity.

This invention teaches the regions of the glycoprotein hormone *beta*-subunit which controls the LH and FSH receptor binding activity. These regions are found between the 10th and 12th cysteine residues. A secondary determinant is also found within a few residues C-terminal of the 12th cysteine. Since the overall structure of the glycoprotein hormones has been conserved during evolution, it is expected that the same regions of all vertebrate gonadotropins are involved in receptor binding specificity. Thus, to obtain analogs with high LH and FSH activity, one would prepare *alpha*, *beta*-heterodimers from the glycoprotein *alpha*-subunit and a *beta*-subunit analog of LH in which the region between the 11th and 12th cysteines was substituted by the residues found between the 11th and 12th cysteines of FSH. To reduce the LH activity of this analog, one would substitute residues between the 10th and 11th cysteines with those found between the 10th and 11th cysteines of FSH. In the case of mammalian hormones, one can also reduce the positive charges and/or increase the negative charges in this region. To reduce the LH activity further in this analog, one would substitute the residues immediately C-terminal of the 12th cysteine with those of FSH. To influence the FSH activity of this analog, one would alter the residues between the 11th and 12th cysteine. One effective strategy in reducing FSH activity is to employ residues from LH. By manipulating the residues between the 10th and 12th cysteines in the LH or CG *beta*-subunits in this way, one can prepare analogs of glycoprotein hormones with nearly any desired receptor binding specificity. In addition, by use of positive charges between the 10th and 11th cysteines, it is possible in mammalian hormone to enhance the binding of the analog to either LH or FSH receptors. Since residues between the 1st and 10th cysteine residues of the *beta*-subunit do not have a great influence on receptor binding specificity, the composition of residues in this region will be of much less importance.

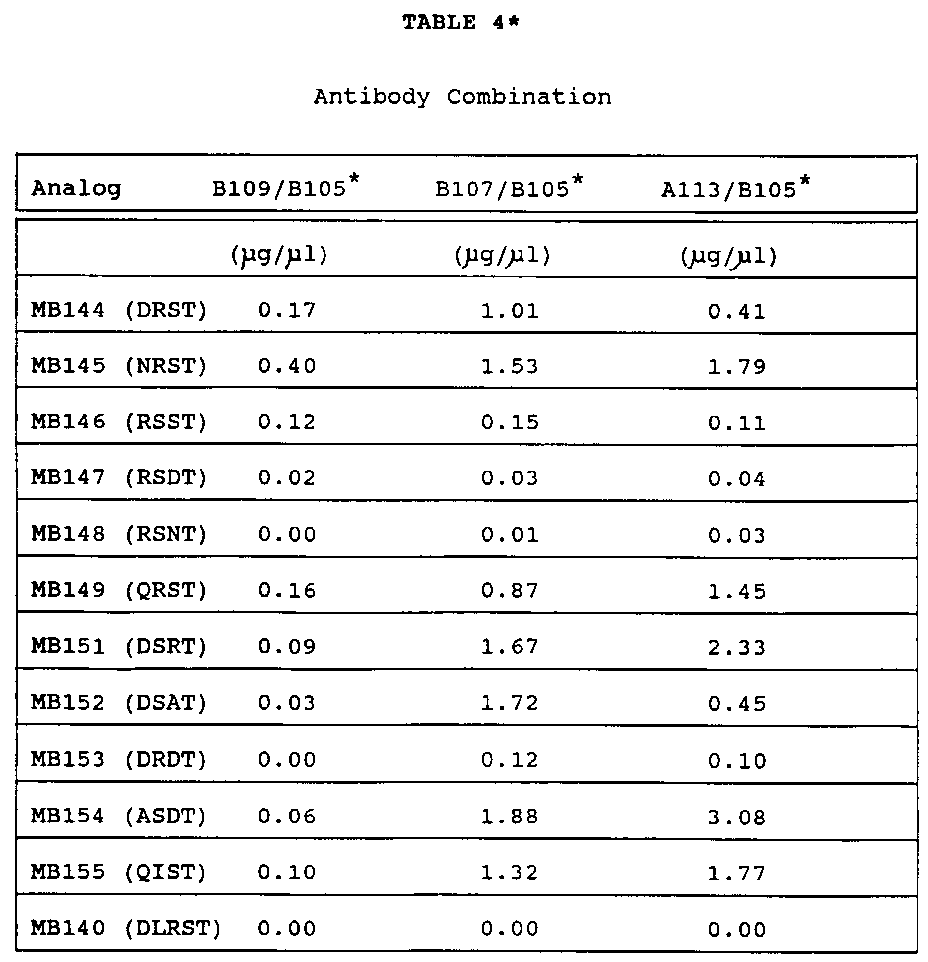

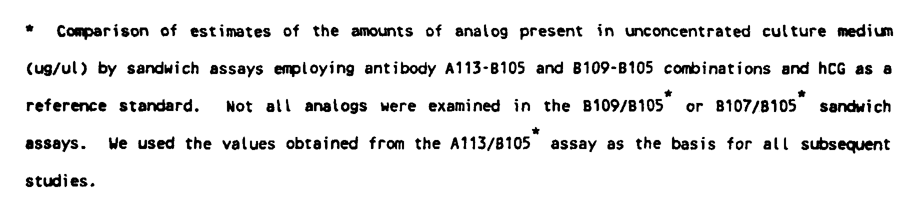

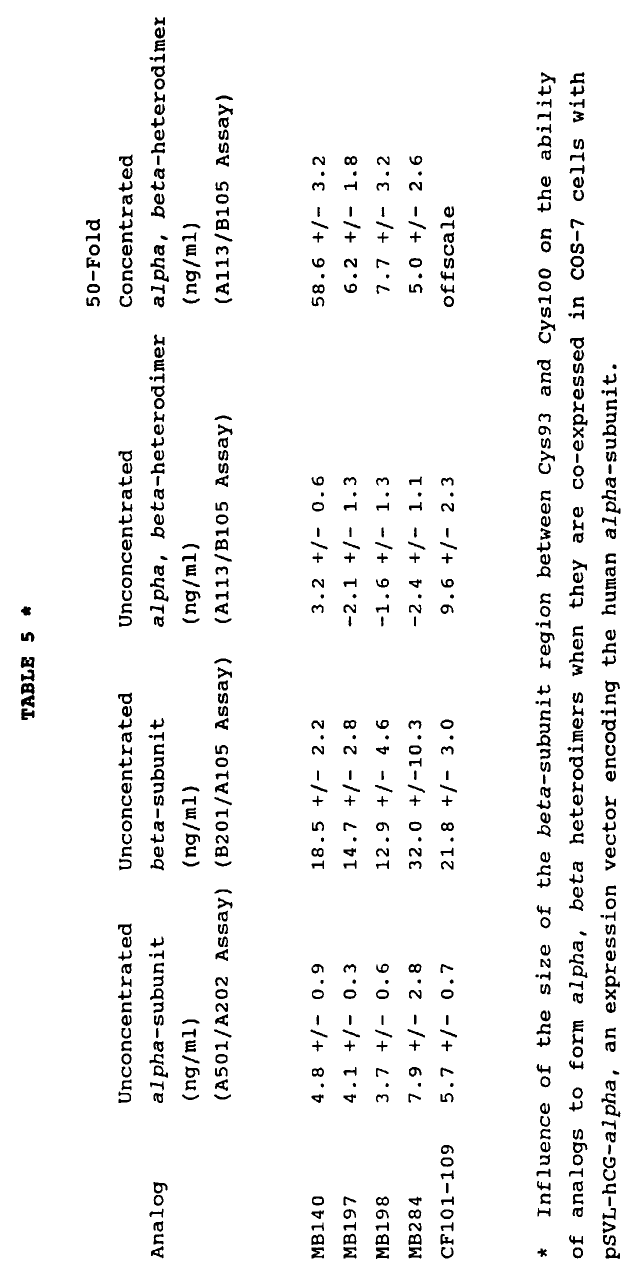

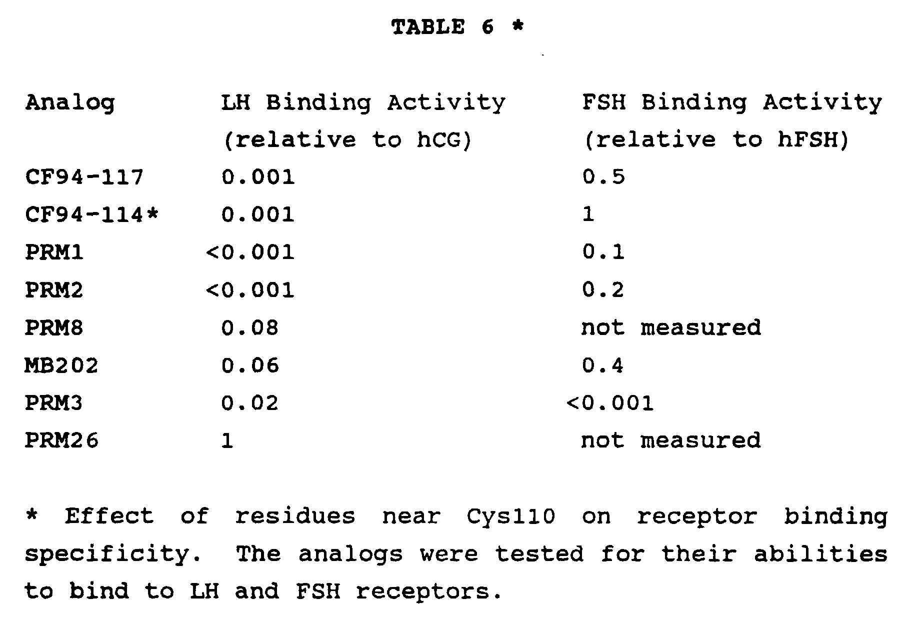

Notes: The values illustrated were determined by measuring the ability of the analogs to inhibit the binding of ¹²⁵I-hCG to intact cells stably expressing LH receptors and the binding of ¹²⁵I-hFSH to intact cells stably expressing FSH receptors. *The values for CF94-114 were taken from our earlier publication and were determined using tissue homogenates (27).

### APPENDIUM OF REFERENCES

1. Yen, S.S.C. and Jaffe, R.B., (1986) "Reproductive Endocrinology: Physiology, Pathophysiology and Clinical Management," 2nd edition, W.B. Saunders Company, Philadelphia (1986).
2. Office of Technology Assessment: Report Brief-Infertility: Medical and Social Choices. Washington, DC, OTA, US Congress, May 1988.
3. Institute of Medicine and National Research Council, Medically assisted conception: an agenda for research. National Academy Press, Washington, DC (1989).
4. Bousfield G.R., and Ward, D.N., (1988) J. Biol. Chem. 263, 12602.
5. Rodgers, N., Mitchess, R., Lambert, A., and Robertson, W.R., (1991) J. Endocrinol. 129 (Supplement), 111.
6. Harlin, J., Kahn, S.A., and Diczfalusy, E., (1986) Fertil. Steril. 46, 1055.
7. Wallach, E.E., (1991) Fertil. Steril. 55, 478.
8. Schenker, J.G. and Weinstein, D., (1978) Fertil. Steril. 30, 255.
9. Murphy, B.D. and Martinuk, S.D., (1991) Endocrine Rev. 12, 27.
10 Campbell, R.K., Dean-Emig, D.M., and Moyle, W.R., (1991) Proc. Nat'l. Acad. Sci. (USA) 88, 760.
11. Pierce, J.G. and Parsons, T.F., (1981) Ann. Rev. Biochem. 50, 465.
12. Fiddes, J.C. and Goodman, H.M., (1980) Nature 286, 684.
13. Fiddes, J.C. and Goodman, H.M., (1979) Nature 281, 351.
14. Keutmann, H.T., Charlesworth, M.C., Mason, K.A., Ostrea, T., Johnson, L., and Ryan, R.J., (1987) Proc. Nat'l. Acad. Sci. (USA) 84, 2038.
15. Santa Coloma, T.A., and Reichert, L.E., Jr., (1990) J. Biol. Chem. 265, 5037.
16. Schneyer, A.L., Sluss, P.M., Huston, J.S., Ridge, R.J., and Reichert. L.E., Jr., (1988) Biochem. 27, 666.
17. Reddy, V.B., Beck, A.K., Garramone, A.J., Vellucci, B., Lustbader, J.W., and Bernsteine, E.G., (1985) Proc. Nat'l. Acad. Sci. (USA) 82, 14204.
18. Matzuk, M.M., Krieger, M., Corless, C.L., and Boime, I., (1987) Proc. Natl. Acad. Sci. (USA) 84, 6354-6358.
19. Matzuk, M.M., Kornmeier, C.M., Whitfield, G.K., Konids, I.A., and Boime, I., (1988) Molec. Endocrinol. 2, 95-100.
20. Matzuk, M.M. and Boime, I., (1988) J. Cell Biol. 106, 1049-1059.
21. Moyle, W.R., Matzuk, M.M., Campbell, R.K., Cogliani, E., Dean-Emig, D.M., Krichevsky, A., Barnett, R.W., and Boime, I., (1990) J. Biol. Chem. 265, 8511.
22. Moyle, W.R., Bahl, O.P., and Marz, L., (1975) J. Biol. Chem. 250, 9163.
23. Sambrook, J., Fritsch, E.F., and Maniatis, T., (1989) Molecular Cloning: A laboratory manual (second edition) Cold Spring Harbor Laboratory Press.
24. Ausubel, F.M., Brent, R., Kingston, R.E. Moore, D.D., Seidman, J.G., Smith, J.A., and Struhl, K., (1987) Current Protocols in Molecular Biology. Greene Publishing Associates and Wiley-Interscience, New York.
25. Moyle, W.R., Anderson, D.M., Macdonald,G.J. and Armstrong, E.G., (1988) J. Receptor Research 8, 419.
26. Bousfield, G. R., W. K. Liu, H. Sugino, and D. N. Ward. 1987. Structural studies on equine glycoprotein hormones. Amino acid sequence of equine lutropin *beta*-subunit. J. Biol. Chem. 262:8610-8620.
27. Campbell, R. K., D. M. Dean Emig, and W. R. Moyle. 1991. Conversion of human choriogonadotropin into a follitropin by protein engineering. Proc. Natl. Acad. Sci. U. S. A. 88:760-764.
28. Moyle, W. R., P. H. Ehrlich, and R. E. Canfield. 1982. Use of monoclonal antibodies to hCG subunits to examine the orientation of hCG in the hormone-receptor complex. Proc. Natl. Acad. Sci. USA 79:2245-2249.
29. Bernard, M. P., R. V. Myers, and W. R. Moyle. 1990. Cloning of rat lutropin (LH) receptor analogs lacking the soybean lectin domain. Mol. Cell. Endocrinol. 71:R19-R23.
30. Moyle, W. R., M. M. Matzuk, R. K. Campbell, E. Cogliani, D. M. Dean Emig, A. Krichevsky, R. W. Barnett, and I. Boime. 1990. Localization of residues that confer antibody binding specificity using human chorionic gonadotropin/luteinizing hormone *beta*-subunit chimeras and mutants. J. Biol. Chem. 265:8511-8518.
31. Jia, X. -C., M. Oikawa, M. Bo, T. Tanaka, T. Ny, I. Boime, and A. J. W. Hsueh. 1991. Expression of human luteinizing hormone (LH) receptor: Interaction with LH and chorionic gonadotropin from human but not equine, rat, and ovine species. Mol. Endocrinol. 5:759-768.
32. Brooker, J., J. F. Harper, W. L. Terasaki, and R. D. Moylan. 1992. Radioimmunoassay of cyclic AMP and cyclic GMP. Adv. Cyclic Nucl. Res. 10:1-33.
33. Sato, G. H., G. Augusti-Tocco, M. Posner, and P. Kelly. 1992. Hormone-secreting and hormone-responsive cell cultures. In Recent Progress in Hormone Research vol 26. E. B. Astwood, editor. Academic Press, New York. 539-546.
34. Pierce, J. G. and T. F. Parsons. 1981. Glycoprotein hormones: structure and function. Ann. Rev. Biochem. 50:465-495.
35. Moyle, W. R. 1980. Biochemistry of gonadotropin receptors. In Oxford Reviews of Reproductive Biology Volume 2. C. A. Finn, editor. Oxford University Press, New York. 123-204.

The invention being thus described, it will be obvious that the same may be varied in many ways. Such variations are not to be regarded as a departure from the scope of the invention and all such modifications are intended to be included within the scope of the following claims.

## Claims

1. An alpha, beta-heterodimeric polypeptide having binding affinity to vertebrate luteinizing hormone (LH) receptors and vertebrate follicle stimulating hormone (FSH) receptors comprising a glycoprotein hormone alpha-subunit polypeptide derived from a vertebrate LH, FSH, TSH or CG and a non-naturally occurring beta-subunit polypeptide, wherein the beta-subunit polypeptide is a chain of amino acids comprising four joined subsequences in the following order:
(a) a first subsequence homologous to the amino acid sequence selected from the group of residues 1-93 of the beta-subunit of a vertebrate chorionic gonadotropin (CG) or lutropin (LH) such as those of the human CG or LH (hCG, hLH), or residues 1-87 of the beta-subunit of a vertebrate follitropin (FSH) such as those of the human FSH (hFSH) or residues 1-88 of the beta subunit of a vertebrate thyrotropin (TSH) such as those of human TSH (hTSH);
(b) a second subsequence homologous to the amino acid sequence of residues 94-97 of the beta-subunit of CG, LH, hCG or hLH or residues 89-92 of the beta-subunit of TSH or hTSH;
(c) a third subsequence homologous to the amino acid sequence of residues 98-100 of the beta-subunit of CG, LH, hCG or hLH or residues 92-94 of the beta-subunit of FSH or hFSH or residues 93-95 of the beta-subunit of TSH or hTSH; and
(d) a fourth subsequence homologous to the amino acid sequence of residues 95-104 of the beta-subunit of FSH or hFSH.

2. The alpha, beta-heterodimeric polypeptide according to claim 1, wherein the glycoprotein hormone alpha-subunit polypeptide is the human alpha-subunit polypeptide.

3. The alpha, beta-heterodimeric polypeptide according to claim 2, wherein the glycoprotein hormone alpha-subunit polypeptide is the alpha-subunit of hCG .

4. The alpha, beta-heterodimeric polypeptide according to claim 2, wherein the glycoprotein hormone alpha-subunit polypeptide is the hLH alpha-subunit polypeptide.

5. The alpha, beta-heterodimeric polypeptide according to claim 1, wherein the first subsequence is homologous to residues 1-93 of the beta-subunit of human chorionic gonadotropin (hCG), the second subsequence is homologous to residues 94-97 of the beta-subunit of human chorionic gonadotropin (hCG), the third subsequence is homologous to residues 98-100 of the beta-subunit of human chorionic gonadotropin (hCG), and the fourth subsequence is homologous to residues 95-104 of the beta-subunit of vertebrate follicle stimulating hormone.

6. A pharmaceutical composition comprising a pharmaceutically acceptable carrier and the alpha, beta-heterodimeric polypeptide according to claim 1.

7. An alpha, beta-heterodimeric polypeptide having greater binding affinity than luteinizing hormone (LH) for vertebrate follicle stimulating hormone (FSH) receptors comprising a glycoprotein hormone alpha-subunit polypeptide derived from a vertebrate LH, FSH, TSH or CG and a non-naturally occurring beta-subunit polypeptide, wherein the beta-subunit polypeptide is a chain of amino acids comprising four joined subsequences in the following order:
(a) a first subsequence homologous to the amino acid sequence selected from the group of residues 1-93 of the beta-subunit of a vertebrate chorionic gonadotropin (CG) or lutropin (LH) such as those of the human CG or LH (hCH, hLH), or residues 1-87 of the beta-subunit of a vertebrate follitropin (FSH) such as those of the human FSH (hFSH) or residues 1-88 of the beta subunit of a vertebrate thyrotropin (TSH) such as those of human TSH (hTSH);
(b) a second subsequence comprising 4 amino acids for residues 94-97 (hCG numbering) not found in hFSH between residues 88-91;
(c) a third sequence comprising 3 amino acids for residues 98-100 (hCG numbering); and
(d) a fourth subsequence homologous to the amino acid sequence of residues 95-104 (FSH numbering) of the beta-subunit of vertebrate follicle stimulating hormone.

8. An alpha, beta-heterodimeric polypeptide having greater binding affinity than luteinizing hormone for follicle stimulating hormone (FSH) receptors comprising a glycoprotein hormone alpha-subunit polypeptide derived from a vertebrate LH, FSH, TSH or CG and a non-naturally occurring beta-subunit polypeptide, wherein the beta-subunit polypeptide is a chimera comprised of amino acids 1-100 of any vertebrate glycoprotein hormone homologous to amino acids found in residues 1-100 of human chorionic gonadotropin and of amino acid residues 101-110 homologous to those of the beta-subunit of FSH or hFSH between amino acid residues 95-104.

9. A process for the preparation of the alpha, beta-heterodimeric polypeptide according to any one of claims 1 to 5, 7 or 8 which comprises producing the subsequences by recombinant or synthetic preparation or by introduction of amino acid sequence variations, and joining the subsequences in the required order.

10. A process for the preparation of the pharmaceutical composition of claim 6 which comprises combining a pharmaceutically acceptable carrier and the alpha, beta-heterodimeric polypeptide according to claim 1.

## Patentansprüche

1. Alpha, beta-heterodimeres Polypeptid mit einer Bindungsaffinität für luteinisierendes Hormon (LH)-Rezeptoren von Vertebraten und follikelstimulierendes Hormon (FSH)-Rezeptoren von Vertebraten, umfassend ein Alpha-Untereinheit-Polypeptid eines Glycoproteinhormons, das von einem Vertebraten-LH, -FSH, -TSH oder -CG stammt, und ein nicht natürlich vorkommendes Beta-Untereinheit-Polypeptid, wobei das Beta-Untereinheit-Polypeptid eine Aminosäurekette ist, die vier verknüpfte Untersequenzen in der folgenden Reihenfolge umfaßt:
a) eine erste Untersequenz, die homolog zur Aminosäuresequenz ist, die ausgewählt ist aus den Resten 1-93 der Beta-Untereinheit eines Choriongonadotropins (CG) oder Lutropins (LH) von Vertebraten, wie solche von menschlichem CG oder LH (hCG, hLH), oder den Resten 1-87 der Beta-Untereinheit eines Vertebraten-Follitropins (FSH), wie solche von menschlichem FSH (hFSH), oder den Resten 1-88 der Beta-Untereinheit eines Vertebraten-Thyrotropins (TSH), wie solche von menschlichem TSH (hTSH);
b) eine zweite Untersequenz, die homolog zu der Aminosäuresequenz der Reste 94 bis 97 der Beta-Untereinheit von CG, LH, hCG oder hLH ist, oder der Reste 89-92 der Beta-Untereinheit von TSH oder hTSH;
c) eine dritte Untersequenz, die homolog ist zu der Aminosäuresequenz der Reste 98-100 der Beta-Untereinheit von CG, LH, hCG oder hLH, oder der Reste 92 bis 94 der Beta-Untereinheit von FSH oder hFSH oder der Reste 93 bis 95 der Beta-Untereinheit von TSH oder hTSH;
d) eine vierte Untersequenz, die homolog zu der Aminosäuresequenz der Reste 95-104 der Beta-Untereinheit von FSH oder hFSH ist.

2. Alpha, beta-heterodimeres Polypeptid nach Anspruch 1, wobei das Alpha-Untereinheit-Polypeptid des Glycoproteinhormons das menschliche Alpha-Untereinheit-Polypeptid ist.

3. Alpha, beta-heterodimeres Polypeptid nach Anspruch 2, wobei das Alpha-Untereinheit-Polypeptid des Glycoproteinhormons die Alpha-Untereinheit von hCG ist.

4. Alpha, beta-heterodimeres Polypeptid nach Anspruch 2, wobei das Alpha-Untereinheit-Polypeptid des Glycoproteinhormons das hLH-Alpha-Untereinheit-Polypeptid ist.

5. Alpha, beta-heterodimeres Polypeptid nach Anspruch 1, wobei die erste Untersequenz homolog ist zu den Resten 1-93 der Beta-Untereinheit von menschlichem Choriongonadotropin (hCG), die zweite Untereinheit homolog ist zu den Resten 94-97 der Beta-Untereinheit von menschlichem Choriongonadotropin (hCG), die dritte Untereinheit homolog ist zu den Resten 98-100 der Beta-Untereinheit von menschlichem Choriongonadotropin (hCG) und die vierte Untereinheit homolog ist zu den Resten 95-104 der Beta-Untereinheit von follikelstimulierendem Hormon von Vertebraten.

6. Arzneimittel, umfassend einen pharmazeutisch verträglichen Träger und das Alpha, beta-heterodimere Polypeptid nach Anspruch 1.

7. Alpha, beta-heterodimeres Polypeptid mit einer größeren Bindungsaffinität als luteinisierendes Hormon (LH) für follikelstimulierendes Hormon (FSH)-Rezeptoren von Vertebraten, umfassend ein Alpha-Untereinheit-Polypeptid eines Glycoproteinhormons, das von einem Vertebraten-LH, -FSH, -TSH oder -CG stammt, und ein nicht natürlich vorkommendes Beta-Untereinheit-Polypeptid, wobei das Beta-Untereinheit-Polypeptid eine Aminosäurekette ist, die vier verknüpfte Untersequenzen in der folgenden Reihenfolge umfaßt:
a) eine erste Untersequenz, die homolog ist zu der Aminosäuresequenz, ausgewählt aus den Resten 1-93 der Beta-Untereinheit eines Choriongonadotropins (CG) oder Lutropins (LH) von Vertebraten, wie solche von menschlichem CG oder LH (hCH, hLH), oder den Resten 1-87 der Beta-Untereinheit des Vertebraten-Follitropins (FSH), wie solche von menschlichem FSH (hFSH), oder den Resten 1-88 der Beta-Untereinheit eines Vertibraten-Thyrotropins (TSH), wie solche von menschlichem TSH (hTSH);
b) eine zweite Untersequenz, die vier Aminosäuren für die Reste 94-97 (hCG-Numerierung) umfaßt, die nicht in hFSH zwischen den Resten 88-91 auftreten;
c) eine dritte Sequenz, die drei Aminosäuren für die Reste 98 bis 100 (hCG-Numerierung) umfaßt;
d) eine vierte Untersequenz, die homolog ist zu der Aminosäuresequenz der Reste 95-104 (FSH-Numerierung) der Beta-Untereinheit von follikelstimulierendem Hormon von Vertebraten.

8. Alpha, beta-heterodimeres Polypeptid mit einer größeren Bindungsaffinität als luteinisierendes Hormon für follikelstimulierendes Hormon (FSH)-Rezeptoren, umfassend ein Alpha-Untereinheit-Polypeptid eines Glycoproteinhormons, das von einen Vertebraten-LH, -FSH, -TSH oder -CG stammt, und ein nicht natürlich vorkommendes Beta-Untereinheit-Polypeptid, wobei das Beta-Untereinheit-Polypeptid eine Chimäre ist, die Aminosäuren 1-100 eines beliebigen Vertebraten-Glycoproteinhormons, die homolog sind zu den Aminosäuren, die in den Resten 1-100 des menschlichen Choriogonatropins vorhanden sind, und Aminosäurereste 101-110 umfaßt, die homolog sind zu denen der Beta-Untereinheit von FSH oder hFSH zwischen den Aminosäureresten 95-104.

9. Verfahren zur Herstellung des Alpha, beta-heterodimeren Polypeptids nach einem der Ansprüche 1-5, 7 oder 8, umfassend die Produktion der Untersequenzen durch rekombinante oder synthetische Herstellung oder durch Einführung von Aminosäuresequenzvariationen, und die Verknüpfung der Untersequenzen in der erforderlichen Reihenfolge.

10. Verfahren zur Herstellung des Arzneimittels nach Anspruch 6, umfassend das Kombinieren eines pharmazeutisch verträglichen Trägers und des Alpha, beta-heterodimeren Polypeptids nach Anspruch 1.

## Revendications

1. Polypeptide alpha,bêta-hétérodimère ayant une affinité de liaison envers les récepteurs de l'hormone lutéinisante de vertébré (LH) et envers les récepteurs de l'hormone de stimulation du follicule de vertébré (FSH) comprenant un polypeptide de sous-unité alpha d'hormone glycoprotéinique provenant de LH, FSH, TSH ou CG de vertébré et un polypeptide de sous-unité bêta d'origine non naturel, dans lequel le polypeptide de sous-unité bêta est une chaîne d'acides aminés comprenant quatre sous-séquences jointes dans l'ordre suivant:
(a) une première sous-séquence homologue à la séquence d'acides aminés choisie dans le groupe des résidus de 1 à 93 de la sous unité bêta de gonatropine chorionique (CG) ou de lutropine (LH) de vertébré telle que celle de la CG ou LH humaine (hCG, hLH), ou des résidus 1 à 87 de la sous-unité bêta de follitropine de vertébré (FSH) telle que celle de la FSH humaine (hFSH) ou les résidus 1 à 88 de la sous-unité bêta de thyrotropine de vertébré (TSH) telle que ceux de TSH humain (hTSH);
(b) une deuxième sous-séquence homologue à la séquence d'acides aminés des résidus 94 à 97 de la sous-unité bêta de CG, LH, hCG ou hLH ou des résidus 89 à 92 de la sous-unité bêta de TSH ou hTSH;
(c) une troisième sous-séquence homologue à la séquence d'acides aminés des résidus 98 à 100 de la sous-unité bêta de CG, LH, hCG ou hLH ou des résidus 92 à 94 de la sous-unité bêta de FSH ou hFSH ou les résidus 93 à 95 de la sous-unité bêta de TSH ou hTSH; et
(d) une quatrième sous-séquence homologue à la séquence d'acides aminés des résidus 95 à 104 de la sous-unité bêta de FSH ou hFSH.

2. Polypeptide alpha,bêta-hétérodimère selon la revendication 1, dans lequel le polypeptide de sous-unité alpha d'hormone glycoprotéinique est le polypeptide de sous-unité alpha humain.

3. Polypeptide alpha,bêta-hétérodimère selon la revendication 2, dans lequel le polypeptide de sous-unité alpha d'hormone glycoprotéinique est la sous-unité alpha de hCG.

4. Polypeptide alpha,bêta-hétérodimère selon la ravendication 2, dans lequel le polypeptide de sous-unité alpha d'hormone glycoprotéinique est le polypeptide de sous-unité alpha hLH.

5. Polypeptide alpha,bêta-hétérodimère selon la revendication 1, dans lequel la première sous-séquence est homologue aux résidus 1 à 93 de la sous-unité bêta de la gonadotropine chorionique humaine (hCG), la deuxième sous-séquence est homologue aux résidus 94 à 97 de la sous-unité bêta de la gonadotropine chorionique humaine (hCG), la troisième sous-séquence est homologue aux résidus 98 à 100 de la sous-unité bêta de la gonadotropine chorionique humaine (hCG) et la quatrième sous-séquence est homologue aux résidus 95 à 104 de la sous-unité bêta de l'hormone de stimulation du follicule de vertébré.

6. Composition pharmaceutique comprenant un véhicule acceptable sur le plan pharmaceutique et le polypeptide alpha,bêta-hétérodimère selon la revendication 1.

7. Polypeptide alpha,bêta-hétérodimère ayant une affinité de liaison plus grande que l'hormone lutéinisante (LH) pour les récepteurs d'hormone de stimulation du follicule de vertébré (FSH) comprenant un polypeptide de sous-unité alpha d'hormone glycoprotéinique provenant d'une LH, FSH, TSH ou CG et un polypeptide de sous-unité bêta d'origine non naturelle, dans lequel le polypeptide de sous-unité bêta est une chaîne d'acides aminés comprenant quatre sous-séquence jointes dans l'ordre suivant:
(a) une première sous-séquence homologue à la séquence d'acides aminés choisie dans le groupe des résidus 1 à 93 de la sous-unité bêta de gonadotropine chorionique (CG) ou de lutropine (LH) de vertébré telle que celle de la CG ou LH humaine ( hCG, hLH), ou des résidus 1 à 87 de la sous-unité bêta d'une follitropine de vertébré (FSH) telle que celle de la FSH humaine (hFSH) ou des résidus 1 à 88 de la sous-unité bêta de thyrotropine de vertébré (TSH) telle que celle de la TSH humaine (hTSH);
(b) une deuxième sous-séquence comprenant quatre acides aminés pour les résidus 94 à 97 (numérotation de hCG) non trouvés dans hFSH entre les résidus 88 à 91;
(c) une troisième séquence comprenant trois acides aminés pour les résidus 98 à 100 (numérotation hCG); et
(d) une quatrième sous-séquence homologue à la séquence d'acides aminés des résidus 95 à 104 (numérotation de FSH) de la sous-unité bêta de l'hormone de stimulation du follicule de vertébré.

8. Polypeptide alpha,bêta-hétérodimère ayant une affinité de liaison plus grande que l'hormone lutéinisante pour les récepteurs de l'hormone de stimulation du follicule (FSH) comprenant un polypeptide de sous-unité alpha d'hormone glycoprotéinique provenant de LH, FSH, TSH ou CG de vertébré et un polypeptide de sous-unité bêta d'origine non naturelle, dans lequel le polypeptide de sous-unité bêta est une chimère constituée des acides aminés 1 à 100 de tout homologue d'hormone glycoptrotéinique de vertébré aux acides aminés trouvés dans les résidus 1 à 100 de la gonadotropine chorionique humaine et des résidus d'acide aminé 101 à 110 homologues à ceux de la sous-unité bêta de FHS ou hFSH entre les résidus d'acide aminé 95 à 104.

9. Procédé pour la préparation du polypeptide alpha, bêta-hétérodimère selon l'une quelconque des revendications 1 à 5, 7 ou 8, qui comprend l'étape consistant à produire les sous-séquence par recombinaison ou préparation de synthèse ou par introduction de variations de séquence d'acides aminés, et à joindre les sous-séquences dans l'ordre requis.

10. Procédé pour la préparation de la composition pharmaceutique de la revendication 6, qui comprend l'étape consistant à combiner un véhicule acceptable sur le plan pharmaceutique et le polypeptide alpha,béta-hétérodimère selon la revendication 1.
